# EUROPEAN PATENT SPECIFICATION

(11) **EP 1 210 372 B1**
(45) Date of publication and mention of the grant of the patent: **23.01.2008**
(21) Application number: 00950679.1
(22) Date of filing: 25.07.2000
(51) Int. Cl.: C07K 16/00

(54) **HUMAN MONOCLONAL ANTIBODIES TO HER2/NEU**
MENSCHLICHE ANTIKÖRPER GEGEN HER2/NEU
ANTICORPS MONOCLONAUX HUMAINS DIRIGES CONTRE HER2/NEU

(30) Priority: 29.07.1999 US 146313 P; 10.03.2000 US 188539 P
(43) Date of publication of application: 05.06.2002
(73) Proprietor: MEDAREX, INC., Annandale New Jersey 08801-0953 (US)
(72) Inventor: KELER, Tibor, Ottsville, PA 18942 (US); DEO, Yashwant, Audubon, PA 19403 (US); VITALE, Laura, Doylestown, PA 18901 (US)
(74) Representative: Lock, Graham James
(86) International application number: PCT/US2000/020272
(87) International publication number: WO 2001/009187

(56) References cited:
- WO-A-94/04679
- WO-A-97/00271
- WO-A-98/02463
- WO-A-99/55367
- CURNOW R T: "CLINICAL EXPERIENCE WITH CD64-DIRECTED IMMUNOTHERAPY. AN OVERVIEW" CANCER IMMUNOLOGY AND IMMUNOTHERAPY,BERLIN,DE, vol. 45, no. 3/04, November 1997 (1997-11), pages 210-215, XP000918956 ISSN: 0340-7004
- BASELGA J ET AL: "PHASE II STUDY OF WEEKLY INTRAVENOUS RECOMBINANT HUMANIZED ANTI-P185HER2 MONOCLONAL ANTIBODY IN PATIENTS WITH HER2/NEU-OVEREXPRESSING METASTATIC BREAST CANCER" JOURNAL OF CLINICAL ONCOLOGY,PHILADELPHIA, PA,US, vol. 14, no. 3, March 1996 (1996-03), pages 737-744, XP000918166
- A JAKOBOVITS: "The long-awaited magic bullets: therapeutic human monoclonal antibodies from transgenic mice" EXPERT OPINION ON INVESTIGATIONAL DRUGS,GB,ASHLEY PUBLICATIONS LTD., LONDON, vol. 7, no. 4, 1 April 1998 (1998-04-01), pages 607-614, XP002084509 ISSN: 1354-3784

## Description

### Background of the Invention

Growth factors and their cognate receptors regulate cell proliferation and differentiation, and alterations in growth factor receptor structure, function and expression have been implicated in the cellular transformation and oncogenesis. Several known proto-oncogenes encode a growth factor or a growth factor receptor, such as c-sis which encodes platelet derived growth factor (PDGF) B chain and c-erbB which encodes the epidermal growth factor (EGF) receptor. The neu oncogene, also known as ERBB2 or HER-2, is implicated in the malignancy of several human adenocarcinomas, especially tumors of the breast and ovary. (1, 2) p185^{HER2} (HER2/neu), the protein product of the c-erbB-2 gene, is a transmembrane protein tyrosine kinase receptor which is homologous to the EGF receptor, and is thought to play are role in the regulation of cell growth and differentiation. Several models suggest that the oncogenic form of p185^{HER2} has a transmembrane mutation with increased ability to dimerize and internalize, as well as increased tyrosine kinase activity. (3, 4, 5)

HER2/neu is a candidate therapeutic target for the treatment of human cancer, especially breast cancer. (6) Antibodies that bind and modulate growth factor receptor function have potential use in immunotherapy, as well as in the diagnosis and prognosis of human tumors. Monoclonal antibodies to HER2 have been developed and demonstrated to have an antitumor effect and to inhibit tumor cell growth in culture and in animal models. (2, 7, 8) For example, in neu transformed cells, cell surface expression of p185^{neu} is required for the maintenance of the transformed phenotype, and *in vivo* treatment with anti-p185^{neu} antibodies leads to the inhibition of tumor growth in mice innoculated with neu transformed cells. (9) Moreover, the treatment of estrogen receptor (ER) positive, HER2/neu positive human breast cancer cells with a combination of tamoxifen and HER2/neu antibody was enhanced compared to either agent alone. (8)

Accordingly, the need exists for developing improved strategies for targeting HER2/neu and for providing safer, less toxic alternatives to the current therapy for human cancer.

WO 97/00271 relates to high affinity human antibodies to tumor antigens. The document discloses human antibodies that specifically bind to c-erbB-2. The document indicates that the antibodies may be used alone or as components of chimeric molecules that specifically target and deliver effector molecules to cells over expressing c-erbB-2.

WO 98/02463 relates to therapeutic multispecific compounds comprised of anti-Fcα receptor antibodies. The document discloses multispecific compounds comprising at least one binding determinant which binds to the Fcχ-receptor on an effector cell. The other binding determinant(s) bind(s) to one or more antigens on a target cell, e.g., the Neu/Her-2 proto-oncogene product or the epidermal growth factor receptor on cancer cells, or to Candida antigens on infected cells. Examples are bispecific and trispecific antibodies. Furthermore, the document discloses use of the multispecific compounds for treatment of cancers or pathogen infections.

J. Clinical Oncology, 14, 737-744, (1996) relates to a phase II study of weekly intravenous recombinant humanized anti-p185^{HER2} monoclonal antibody in patients with HER2/neu -- overexpressing metastatic breast cancer. The document discloses that the recombinant humanized monoclonal antibody (rhuMAb) HER2 is well tolerated and clinically active in patients with HER2 - overexpressing metastatic breast cancers that had received extensive prior therapy. The document concludes that this is evidence that targeting growth factor receptors can cause regression of human cancer and justifies further evaluation of this agent.

Exp. Opin. Invest. Drugs, Vol. 7, 607-614, (1998) relates to therapeutic human monoclonal antibodies from transgenic mice. The document discloses the general advantages of fully human high affinity antibodies produced in transgenic mice, such as reduced immunogenicity. Examples of human antibodies against human antigens are indicated, including a human anti-EGF receptor antibody.

WO 99/55367 relates to internalizing erbB2 antibodies. The document discloses erbB2-binding internalizing antibodies. The antibodies, designated F5 and C1, specifically bind to c-erbB2 antigen and, upon binding, are readily internalized into the cell bearing the c-erbB2 marker. Chimeric molecules comprising the F5 and/or C1 antibodies attached to one or more effector molecules are disclosed.

### Summary of the Invention

The present invention provides isolated human monoclonal antibodies or antibody fragments as defined in the appended claims. The invention also provides compositions containing one or a combination of such antibodies, an immunoconjugate comprising the human antibody or antibody fragment, a composition comprising the immunoconjugate, a bispecific molecule comprising the human antibody or antibody fragment and an additional binding specificity for an Fc receptor, in vitro uses and uses in the manufacture of medicaments for inhibiting growth of a tumor cell expressing HER2/neu, and for treatment of adenocarcinomas e.g., salivary gland, stomach and kidney carcinomas, mammary gland carcinoma, lung carcinoma, squamous cell carcinoma, and ovarian cancer. In certain embodiments, the human antibody binds to the HER2/neu receptor with high affinity, and inhibits the growth and/or mediating cell killing of cells expressing HER2/neu (*in vitro* and *in vivo*) in the presence of human effector cells. Accordingly, the human monoclonal antibodies of the invention can be used as diagnostic or therapeutic agents *in vivo* and *in vitro.*

Isolated human antibodies of the invention encompass various antibody isotypes, such as IgG1, IgG2, IgG3, IgG4, IgM, IgA1, IgA2, IgAsec, IgD, and IgE. Typically, they include IgG1 (*e.g.,* IgG1k) and IgM isotypes. The antibodies can be full-length (*e.g.,* an IgG1 or IgG4 antibody) or can include only an antigen-binding portion (*e.g.,* a Fab, F(ab')₂, Fv or a single chain Fv fragment). In one embodiment, the human antibodies are recombinant human antibodies. In another embodiment, the human antibodies arc produced by a hybridoma which includes a B cell obtained from a transgenic non-human animal, e.g. a transgenic mouse, having a genome comprising a human heavy chain transgene and a human light chain transgene fused to an immortalized cell. In particular embodiments, the antibodies arc produced by hybridomas referred to herein as 3.F2, 1.D2, 2.E8, 1.B10 and 3.B4.

Human anti-HER2/neu antibodies of the present invention have one or more of the following properties:
a) specificity for the HER2/neu receptor;
b) a binding affinity to HER2/neu with an affinity constant of at least about 10⁷ M⁻¹, preferably about 10⁹ M⁻¹, and more preferably, about 10¹⁰ M⁻¹ to 10¹¹ M⁻¹ or higher;
c) an association constant (K_{assoc}) with HER2/neu of at least about 10³, more preferably about 10⁴ and most preferably about 10⁵ M⁻¹S⁻¹;
d) a dissociation constant (K_{dis}) from HER2/neu of about 10⁻³ s⁻¹, preferably about 10⁻⁴ s⁻¹, more preferably, 10⁻⁵s⁻¹, and most preferably, 10⁻⁶s⁻¹;
e) the ability to opsonize a cell expressing HER2/neu;
f) the ability to inhibit growth and/or mediate phagocytosis and killing of cells expressing HER2/neu (e.g., a tumor cell) in the presence of human effector cells at a concentration of about 10 µg/ml or less (e.g., *in vitro*); or
g) the ability to be internalized by HER2/neu expressing cells after binding to HER2/neu.

Examples of tumor cells which can be targeted by the human antibodies of the invention include, but are not limited to: an adenocarcinoma cell, e.g. salivary gland, stomach and kidney, a mammary gland carcinoma cell, a lung carcinoma cell, a squamous cell carcinoma cell, and an ovarian cancer cell.

In one embodiment, isolated human antibodies of the invention bind to the HER2/neu antigen with an affinity constant of at least about 10⁷ M⁻¹, preferably about 10⁸ M⁻¹, more preferably, about 10⁹ M⁻¹, and more preferably about 10¹⁰ to 10¹¹ M⁻¹ or stronger, and are capable of inhibiting growth and/or mediating phagocytosis and killing of cells expressing HER2/neu by human effector cells, e.g., polymorphonuclear cells (PMNs) or IFN-γ induced macrophages, with an IC₅₀ of about 1 x 10⁻⁷ M or less, or at a concentration of about 10 µg/ml or less *in vitro.* In certain embodiments, a human anti-HER2/neu antibody of the invention inhibits growth and/or mediating cytolysis and killing of cells expressing HER2/neu at a concentration of about 5-10µg/ml, preferably about 1-5 µg/ml, or more preferably about 0.5-1 µg/ml *in vitro.*

Nucleic acid molecules encoding the antibodies, or antigen-binding portions, of the invention can be provided. Accordingly, recombinant expression vectors which include the antibody-encoding nucleic acids, and host cells transfected with such vectors, are described herein, as are methods of making the antibodies of the invention by culturing these host cells.

Isolated B-cells can be provided from a transgenic non-human animal, e.g., a transgenic mouse, which are capable of expressing various isotypes (e.g., IgG, IgA and/or IgM) of human monoclonal antibodies that specifically bind to HER2/neu. Preferably, the isolated B cells are obtained from a transgenic non-human animal, e.g., a transgenic mouse, which has been immunized with a purified or enriched preparation of HER2/neu antigen and/or cells expressing the HER2/neu receptor. Preferably, the transgenic non-human animal e.g. a transgenic mouse, has a genome comprising a human heavy chain transgene and a human light chain transgene. The isolated B-cells are then immortalized to provide a source (e.g. a hybridoma) of human monoclonal antibodies to HER2/neu.

A hybridoma can be provided capable of producing human monoclonal antibodies that specifically bind to HER2/neu. The hybridoma may include a B cell obtained from a transgenic non-human animal, e.g., a transgenic mouse, having a genome comprising a human heavy chain transgene and a human light chain transgene fused to an immortalized cell. The transgenic non-human animal can be immunized with a purified or enriched preparation of HER2/neu antigen and/or cells expressing the HER2/neu receptor to generate antibody-producing hybridomas. Particular hybridomas include 3.F2, 1.D2, 2.E8, 1.B10 and 3.B4.

A transgenic non-human animal can be provided, such as a transgenic mouse (also referred to herein as "HuMab"), which express human monoclonal antibodies that specifically bind to HER2/neu. The transgenic non-human animal may be a transgenic mouse having a genome comprising a human heavy chain transgene and a human light chain transgene. The transgenic non-human animal can be immunized with a purified or enriched preparation of HER2/neu antigen and/or cells expressing the HER2/neu receptor. Preferably, the transgenic non-human animal, e.g., the transgenic mouse, is capable of producing multiple isotypes of human monoclonal antibodies to the HER2/neu receptor (e.g., IgG, IgA, and/or IgM) by undergoing V-D-J recombination and isotype switching. Isotype switching may occur by, e.g., classical or non-classical isotype switching.

Methods can be provided for producing human monoclonal antibodies which specifically react with HER2/neu. The method may include immunizing a transgenic non-human animal, e.g. a transgenic mouse, having a genome comprising a human heavy chain transgene and a human light chain transgene, with a purified or enriched preparation of HER2/neu antigen and/or cells expressing the HER2/neu receptor. B cells (e.g., splenic B cells) of the animal are then obtained and fused with myeloma cells to form immortal, hybridoma cells that secrete human monoclonal antibodies against HER2/neu.

Isolated anti-HER2/neu human monoclonal antibodies of the invention, or antigen binding portions thereof, can be derivatized or linked to another functional molecule, e.g., another peptide or protein (e.g., an Fab' fragment or ScFv antibody, or a tumor ligand). For example, an antibody or antigen-binding portion of the invention can be functionally linked (e.g., by chemical coupling, genetic fusion, noncovalent association or otherwise) to one or more other molecular entities, such as another antibody (*e.g.*, a bispecific or a multispecific antibody). Accordingly, in another aspect, the present invention features a bispecific or multispecific molecule comprising at least one first binding specificity for HER2/neu and a second binding specificity an Fc receptor, e.g., human FcγRI (CD64) or a human Fcα receptor (CD89). In one embodiment, these bi- and multispecific molecules further include a tumor ligand, such as EGF. Accordingly, multispecific molecules of the invention also include trispecific, tetraspecific and other multispecific molecules.

In a particular embodiment, bispecific and multispecific molecules of the invention comprise at least one human antibody, or fragment thereof (e.g., an Fab, Fab', F(ab')₂, Fv, or a single chain Fv). Such bi- and trispecific molecules can include multiple human antibody portions, or fragments thereof (e.g., an Fab, Fab', F(ab')₂, Fv, or a single chain Fv), and/or can also include "chimeric" or a "humanized" antibody portions or fragments (e.g., a "humanized" antibody that has a variable region, or at least a complementarity determining region (CDR), derived from a non-human antibody (e.g., murine) with the remaining portion(s) being human in origin).

In general, bi- and multispecific molecules of the invention include at least one antibody or fragment thereof that binds to an Fc receptor, such as a human IgG receptor, i.e., an Fc-gamma receptor (FcγR), such as FcγRI (CD64), FcγRII(CD32), and FcγRIII (CD16), or a human FcαR (CD89). Preferred Fc receptors include the high affinity FcγRI receptor and FcαR receptor (CD89). FcαRI and FcγRI are preferred trigger receptors for use in the invention because they are (1) expressed primarily on immune effector cells, e.g., monocytes, PMNs, macrophages and dendritic cells; (2) expressed at high levels (e.g., 5,000-100,000 per cell); (3) mediators of cytotoxic activities (e.g., ADCC, phagocytosis); (4) mediate enhanced antigen presentation of antigens, including self-antigens, targeted to them. In a preferred embodiment, the bispecific and multispecific molecules bind to an Fc receptor at a site which is distinct from the immunoglobulin (e.g., IgG or IgA) binding site of the receptor. Therefore, the binding of the bispecific and multispecific molecules is not blocked by physiological levels of immunoglobulins.

In another aspect, the present invention features a human anti-HER2/neu antibody, or a fragment thereof, conjugated to a therapeutic moiety, e.g., a cytotoxic drug, an enzymatically active toxin, or a fragment thereof, a radioisotope, or a small molecule anti-cancer drug.

Target-specific effector cells can be provided which comprise an effector cell expressing an Fc receptor, e,g., a macrophage or an activated PMN cell, and a bispecific or multispecific molecule of the invention.

In another aspect, the present invention provides compositions, e.g., pharmaceutical and diagnostic compositions, comprising a pharmaceutically acceptable carrier and at least one human monoclonal antibody of the invention, or an antigen-binding portion thereof, which specifically binds to HER2/neu. In one embodiment, the composition comprises a combination of the human antibodies or antigen-binding portions thereof, preferably each of which binds to a distinct epitope. For example, a pharmaceutical composition comprising a human monoclonal antibody that mediates highly effective killing of target cells in the presence of effector cells can be combined with another human monoclonal antibody that inhibits the growth of cells expressing HER2/neu. Thus, the combination provides multiple therapies tailored to provide the maximum therapeutic benefit. Compositions, e.g., pharmaceutical compositions, comprising a combination of at least one human monoclonal antibody of the invention, or antigen-binding portions thereof, and at least one bispecific or multispecific molecule of the invention, are also within the scope of the invention.

A method can be provided for inhibiting the proliferation and/or differentiation of a cell expressing HER2/neu by inhibiting growth and/or by inducing phagocytosis and/or killing of target cells by human effector cells, such as human polymorphonuclear cells (PMNs), using an antibody, or antigen-binding portion thereof (or a bispecific or multispecific antibody) of the invention.

The method may comprise contacting a cell expressing the HER2/neu receptor either *in vitro* or *in vivo* with one or a combination of human monoclonal antibodies of the invention, or an antigen-binding portion thereof, in the presence of a human effector cell. The method can be employed in culture, e.g. *in vitro* or *ex vivo* (e.g., cultures comprising cells expressing the HER2/neu receptor and effector cells). For example, a sample containing cells expressing the HER2/neu receptor and effector cells can be cultured *in vitro,* and combined with an antibody of the invention, or an antigen-binding portion thereof (or a bispecific or multispecific molecule of the invention). Alternatively, the method can be performed in a subject, e.g., as part of an *in vivo* (e.g., therapeutic or prophylactic) protocol.

For *in vivo* methods, the antibody, or antigen-binding portion thereof (or a bispecific or multispecific molecule of the invention), can be administered to a human subject suffering from a HER2/neu-related disease such that growth inhibition, phagocytosis and/or killing of cells expressing the HER2/neu receptor is induced.

The subject can be additionally treated with an agent that modulates, e.g., enhances or inhibits, the expression or activity of Fc receptor, e.g., an Fcα receptor or an Fcγ receptor, by for example, treating the subject with a cytokine. Preferred cytokines for administration during treatment with the bispecific and multispecific molecule include of granulocyte colony-stimulating factor (G-CSF), granulocyte-macrophage colony-stimulating factor (GM-CSF), interferon-γ (IFN-γ), and tumor necrosis factor (TNF).

Isolated human monoclonal antibody compositions of the invention also can be administered in combination with other known therapies, e.g., an anti-cancer therapy

Exemplary diseases that can be treated (e.g., ameliorated) or prevented using the compositions of the invention include, but are not limited to, tumorigenic diseases. Such tumorigenic diseases include an adenocarcinoma, e.g. salivary gland, stomach and kidney, mammary gland carcinoma, lung carcinoma, squamous cell carcinoma, and ovarian cancer.

A method can be provided for detecting *in vitro* or *in vivo* the presence of the HER2/neu antigen in a sample, e.g., for diagnosing a HER2/neu-related disease. In one embodiment, this is achieved by contacting a sample to be tested, along with a control sample, with a human monoclonal antibody of the invention, or an antigen-binding portion thereof (or a bispecific or multispecific molecule), under conditions that allow for formation of a complex between the antibody and HER2/neu. Complex formation is then detected (e.g., using an ELISA) in both samples, and any statistically significant difference in the formation of complexes between the samples is indicative the presence of the HER2/neu antigen in the test sample.

Other features and advantages of the instant invention be apparent from the following detailed description and claims.

### Brief Description of the Drawings

*Figure 1* is a schematic diagram of the targeted insertion of a neo cassette into the SmaI site of the µl exon. *Panel A* depicts the genomic structure of the µ locus, where the filled boxes represent the µ exons. *Panel B* is a schematic diagram of the CmD targeting vector. *Panel C* depicts the targeted µ locus in which the neo cassette has been inserted into µl.
*Figure 2, panel A* is a bar graph depicting the binding of anti-HER2/neu antibodies from three hybridoma cultures, 1.D2, 2.E8, and 3.F2, to recombinant HER2/neu, as measured by ELISA; *panel B* is a graph depicting the binding of purified anti-HER2/neu antibodies 3.F2, 2.E8, 1.D2, 1.B10, and 3.B4 to HER2/neu antigen shed from tumor cells, as measured by ELISA.
*Figure 3, panels A-C,* are graphs comparing the binding of purified human anti-HER2/neu monoclonal antibodies (1.D2, 2.E8, 3.F2, 1.B10 and 3.B4) to SKBR-3 (*A and B*) and BT-474 (*C*) tumor cells, as detected by flow cytometry.
*Figure 4* is a graph comparing the binding of purified human anti-HER2/neu monoclonal antibody F(ab')₂ fragments (3.F2 and 2.E8) to tumor cells (SKBR-3, BT-474 and A431), as detected by flow cytometry.
*Figure 5, panels A and B,* are graphs depicting the inhibition of SKBR-3 tumor cell growth by purified human anti-HER2/neu monoclonal (1.D2, 2.E8, 3.F2, 1.B10 and 3.B4), as measured by cell density.
*Figure 6* is a graph depicting the inhibition of SKBR-3 tumor cell growth by purified human anti-HER2/neu monoclonal antibody F(ab')₂ fragments (3.F2 and 2.E8), as measured by cell density.
*Figure 7, panels A and B,* are graphs depicting the inhibition of BT-474 tumor cell growth by purified human anti-HER2/neu monoclonal antibodies (1.D2, 1.B10, 3.B4, 3.F2 and 2.E8) and their F(ab')₂ fragments, as measured by cell density.
*Figure 8, panels A and B,* are graphs depicting the antibody dependent cytolysis of SKBR-3 tumor cells by mononuclear cells mediated by purified human anti-HER2/neu monoclonal antibodies (1.D2, 2.E8, and 3.F2) (*A*); or by IFN-γ induced macrophages in the presence of purified human anti-HER2/neu antibodies (3.F2, 2.E8, 1.D2, 1.B10, and 3.B4) (*B*), as measured by ⁵¹Cr release.
*Figure 9* is a graph depicting the antibody dependent cytolysis of SKBR-3 and BT-474 tumor cells by polymorphonuclear cells mediated by a human anti-HER2/neu × anti-CD89 bispecific molecule (14.1 × 3.F2), as measured by ⁵¹Cr release.
*Figure 10* is a graph depicting the antibody dependent cytolysis of SKBR-3 and BT-474 tumor cells by monocytes mediated by a human anti-HER2/neu × anti-CD89 bispecific molecule (14.1 × 3.F2), as measured by ⁵¹Cr release.
*Figure 11* is a graph depicting the antibody dependent cytolysis of BT-474 tumor cells by whole blood mediated by a human anti-HER2/neu × anti-CD89 bispecific molecule (14.1 × 3.F2), as measured by ⁵¹Cr release.
*Figures 12* is a schematic representation of bispecific single chain fusion molecules, 931 and 934, and their parent HuMabs. The variable light and heavy chain regions of 14.1 and 3F2 were used to generate the 931 and 934 (w/EGF) constructs.
*Figure* 13 is a schematic representation of a chemically conjugated bispecific molecule, made up of the the fab' fragments of 14.1 and 3F2 (anti-HER2), used as positive control.

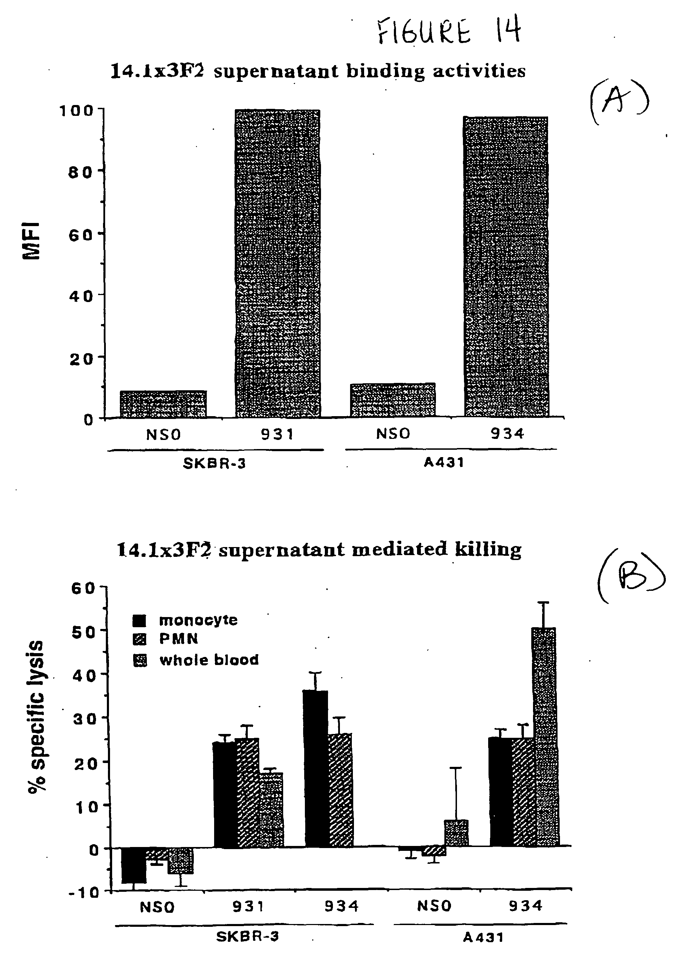

*Figure 14(a)* is a bar graph showing screening (binding) assays for fusion proteins 931 and 934 expressed in transfectoma supernatants as measured by flow cytometry analysis. Transfected (931 & 934) and untransfected (NSO) supernatant was incubated with either SKBR-3 or A431 tumor cell lines. Binding of fusion protein was detected by staining with goat anti-human IgG fab-2 conjugated to phycoerythrin. Figure 14(b) is a bar grapg showing ADCC analysis of fusion proteins 931 and 934. Chromium release assays were done with an incubation period of 16-18 h and an effector (monocytes, PMN) to target (SKBR-3, A431) ration of 100:1. Tumor cell killing was detected using transfected (931 & 934) and untransfected (NSO) supernatant to mediate specific lysis.

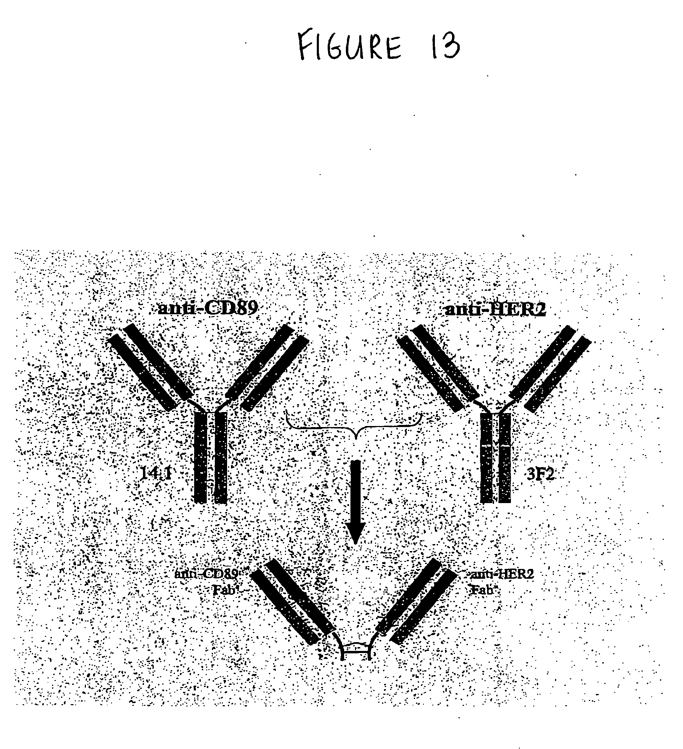

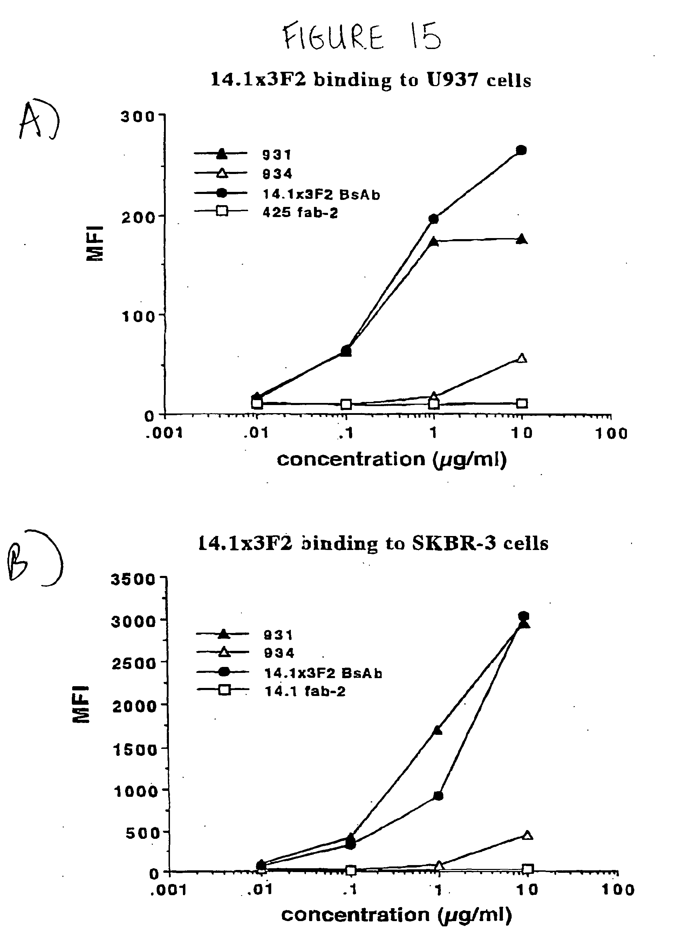

*Figure 15(a)* is a graph showing binding (as measured by flow cytometry) of single-chain fusion proteins 931 and 934 to U937 cells. An fab' fragment of 425, an anti-EGF-R mAb which does not bind U937 cells, was used as a negative control. The chemically linked bispecific (14.1 x 3F2) shown in Figure 13 was used as a positive control. Figure 15(a) is a graph showing binding (as measured by flow cytometry) of single-chain fusion proteins 931 and 934 to SKBR-3 cells. 14.1 fab-2 was used as a negative control. The chemically linked bispecific (14.1 x 3F2) shown in Figure 13 was again used as a positive control.
*Figure 16* is a graph showing binding (as measured by flow cytometry) of single-chain fusion protein 934 to A431 cells. This experiment was done the same way as shown in Figure 15, except that A431 tumor cells which over-express the EGF-R were used. A fusion protein consisting of the sFv fragment of 14.1 fused to EGF was used as a positive control, and 14.1 fab-2 as a negative control.
*Figure 17* is a graph showing PMN (effector cell)-mediated cytotoxicity of SKBR3 (Figure 17(a)) and BT474 (Figure 17(b)) tumor cells by single-chain fusion protein 931. Chromium release assays were done with an incubation period of 16-18 h and an effector to target ration of 100:1. 14.1 fab-2 was used as negative control in each experiment.
*Figure 18* is a graph showing monocyte (effector cell)-mediated cytotoxicity of SKBR3 (Figure 18(a)) and BT474 (Figure 18(b)) tumor cells by single-chain fusion protein 931. Chromium release assays were done with an incubation period of 16-18 h and an effector to target ration of 100:1. 14.1 fab-2 was used as negative control in each experiment.

### Detailed Description of the Invention

The present invention provides novel human antibody and antibody fragments for treating and diagnosing diseases characterized by expression, particularly over-expression, of HER2/neu growth factor receptor (referred to herein as "HER2/neu") and/or related receptors. The human antibodies can be produced in a non-human transgenic animal, e.g., a transgenic mouse, capable of producing multiple isotypes of human monoclonal antibodies to HER2/neu (e.g., IgG, IgA and/or IgE) by undergoing V-D-J recombination and isotype switching. Accordingly, various aspects of the invention include antibodies and antibody fragments, and pharmaceutical compositions thereof. Methods of using the antibodies of the invention can be provided to detect a cell expressing HER2/neu or a related, cross-reactive growth factor receptor, or to inhibit growth, differentiation and/or motility of a cell expressing HER2/neu, either *in vitro* or *in vivo.*

In order that the present invention may be more readily understood, certain terms are first defined. Additional definitions are set forth throughout the detailed description.

The terms "HER2/neu," "HER2," "HER2/neu antigen" and "HER2/neu receptor" are all used interchangeably herein, and include any variants or isoforms of the human HER2/neu receptor, encoded by the human *erbB-2* gene (also known as the *HER2* gene), which naturally occur on the surface of cells, e.g. a human tumor cell. As used herein, the term "*neu*," "*neu* antigen" and "*neu* receptor" refer to the rat equivalent of human HER2/neu, encoded by the rat *erbB-2* gene (also referred to as the rat *neu* gene). In a preferred embodiment, binding of an antibody of the invention to the HER2/neu-antigen inhibits the growth of cells expressing HER2/neu (e.g., a tumor cell). In another preferred embodiment, binding of an antibody of the invention to the HER2/neu-antigen mediates effector cell phagocytosis and/or killing of cells expressing HER2/neu.

As used herein, the term "antibody" refers to a glycoprotein comprising at least two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen-binding portion" of an antibody (or simply "antibody portion"), as used herein, refers to one or more fragments of an antibody that retain the ability to specifically bind to an antigen (*e.g.*, HER2/neu). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

The term "bispecific molecule" is intended to include any agent, e.g., a protein, peptide, or protein or peptide complex, which has two different binding specificities which bind to, or interact with (a) a cell surface antigen and (b) an Fc receptor on the surface of an effector cell. The term "multispecific molecule" or "heterospecific molecule" is intended to include any agent, e.g., a protein, peptide, or protein or peptide complex, which has more than two different binding specificities which bind to, or interact with (a) a cell surface antigen, (b) an Fc receptor on the surface of an effector cell, and (c) at least one other component. Accordingly, the invention includes, but is not limited to, bispecific, trispecific, tetraspecific, and other multispecific molecules which are directed to cell surface antigens, such as HER2/neu, and to Fc receptors on effector cells. The term "bispecific antibodies" further includes diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad Sci. USA 90:6444-6448; Poljak, R.J., et al. (1994) Structure 2:1121-1123).

As used herein, the term "heteroantibodies" refers to two or more antibodies, antibody binding fragments (e.g., Fab), derivatives therefrom, or antigen binding regions linked together, at least two of which have different specificities. These different specificities include a binding specificity for an Fc receptor on an effector cell, and a binding specificity for an antigen or epitope on a target cell, e.g., a tumor cell. The term "human antibody", as used herein, is intended to include antibodies having variable and constant regions derived from human germline immunoglobulin sequences. The human antibodies of the invention may include amino acid residues not encoded by human germline immunoglobulin sequences (*e.g.*, mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

The terms "monoclonal antibody" or "monoclonal antibody composition" as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g., a transgenic mouse, having a genome comprising a human heavy chain transgene and a light chain transgene fused to an immortalized cell.

The term "recombinant human antibody", as used herein, is intended to include all human antibodies that are prepared, expressed, created or isolated by recombinant means, such as antibodies isolated from an animal (*e.g*., a mouse) that is transgenic for human immunoglobulin genes (described further in Section I, below); antibodies expressed using a recombinant expression vector transfected into a host cell, antibodies isolated from a recombinant, combinatorial human antibody library, or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

As used herein, a "heterologous antibody" is defined in relation to the transgenic non-human organism producing such an antibody. This term refers to an antibody having an amino acid sequence or an encoding nucleic acid sequence corresponding to that found in an organism not consisting of the transgenic non-human animal, and generally from a species other than that of the transgenic non-human animal.

As used herein, a "heterohybrid antibody" refers to an antibody having a light and heavy chains of different organismal origins. For example, an antibody having a human heavy chain associated with a murine light chain is a heterohybrid antibody. Examples of heterohybrid antibodies include chimeric and humanized antibodies, discussed supra.

An "isolated antibody", as used herein, is intended to refer to an antibody which is substantially free of other antibodies having different antigenic specificities (e.g., an isolated antibody that specifically binds to HER2/neu is substantially free of antibodies that specifically bind antigens other than HER2/neu). An isolated antibody that specifically binds to an epitope, isoform or variant of human HER2/neu may, however, have cross-reactivity to other related growth factor receptors, e.g., from other species (e.g., HER2/neu species homologs). Moreover, an isolated antibody may be substantially free of other cellular material and/or chemicals. In one embodiment of the invention, a combination of "isolated" monoclonal antibodies having different specificities are combined in a well defined composition.

As used herein, "specific binding" refers to antibody binding to a predetermined antigen. Typically, the antibody binds with an affinity of at least about 1 x 10⁷ M⁻¹, and binds to the predetermined antigen with an affinity that is at least two-fold greater than its affinity for binding to a non-specific antigen (e.g., BSA, casein) other than the predetermined antigen or a closely-related antigen. The phrases "an antibody recognizing an antigen" and " an antibody specific for an antigen" are used interchangeably herein with the term "an antibody which binds specifically to an antigen".

As used herein, the term "high affinity"for an IgG antibody refers to a binding affinity of at least about 10⁷M⁻¹, preferably at least about 10⁹M⁻¹, more preferably at least about 10¹⁰M⁻¹, 10¹¹M⁻¹, 10¹²M⁻¹ or greater, e.g., up to 10¹³M⁻¹ or greater. However, "high affinity" binding can vary for other antibody isotypes. For example, "high affinity" binding for an IgM isotype refers to a binding affinity of at least about 1 x 10⁷M⁻¹.

The term "K_{assoc}", as used herein, is intended to refer to the association constant of a particular antibody-antigen interaction.

The term "K_{dis}", as used herein, is intended to refer to the dissociation constant of a particular antibody-antigen interaction.

As used herein, "isotype" refers to the antibody class (e.g., IgM or IgGl) that is encoded by heavy chain constant region genes.

As used herein, "isotype switching" refers to the phenomenon by which the class, or isotype, of an antibody changes from one 1g class to one of the other Ig classes.

As used herein, "nonswitched isotype" refers to the isotypic class of heavy chain that is produced when no isotype switching has taken place; the CH gene encoding the nonswitched isotype is typically the first CH gene immediately downstream from the functionally rearranged VDJ gene. Isotype switching has been classified as classical or non-classical isotype switching. Classical isotype switching occurs by recombination events which involve at least one switch sequence region in the transgene. Non-classical isotype switching may occur by, for example, homologous recombination between human σ_{µ} and human Σ_{µ} (δ-associated deletion). Alternative non-classical switching mechanisms, such as intertransgene and/or interchromosomal recombination, among others, may occur and effectuate isotype switching.

As used herein, the term "switch sequence" refers to those DNA sequences responsible for switch recombination. A "switch donor" sequence, typically a µ switch region, will be 5' (i.e., upstream) of the construct region to be deleted during the switch recombination. The "switch acceptor" region will be between the construct region to be deleted and the replacement constant region (e.g., γ, ε, etc.). As there is no specific site where recombination always occurs, the final gene sequence will typically not be predictable from the construct.

As used herein, "glycosylation pattern" is defined as the pattern of carbohydrate units that are covalently attached to a protein, more specifically to an immunoglobulin protein. A glycosylation pattern of a heterologous antibody can be characterized as being substantially similar to glycosylation patterns which occur naturally on antibodies produced by the species of the nonhuman transgenic animal, when one of ordinary skill in the art would recognize the glycosylation pattern of the heterologous antibody as being more similar to said pattern of glycosylation in the species of the nonhuman transgenic animal than to the species from which the CH genes of the transgene were derived.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

The term "rearranged" as used herein refers to a configuration of a heavy chain or light chain immunoglobulin locus wherein a V segment is positioned immediately adjacent to a D-J or J segment in a conformation encoding essentially a complete VH or VL domain, respectively. A rearranged immunoglobulin gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

The term "nucleic acid molecule", as used herein, is intended to include DNA molecules and RNA molecules. A nucleic acid molecule may be single-stranded or double-stranded, but preferably is double-stranded DNA.

The term "isolated nucleic acid molecule", as used herein in reference to nucleic acids encoding antibodies or antibody portions (e.g., VH, VL, CDR3) that bind to HER2/neu, is intended to refer to a nucleic acid molecule in which the nucleotide sequences encoding the antibody or antibody portion are free of other nucleotide sequences encoding antibodies or antibody portions that bind antigens other than HER2/neu, which other sequences may naturally flank the nucleic acid in human genomic DNA. SEQ ID NOS: 1-12 correspond to the nucleotide and amino acid sequences comprising the heavy chain (VH) and light chain (VL) variable regions of the 3.F2, 1.D2 and 2.E8 human anti-HER2/neu monoclonal antibodies of the invention. In particular, SEQ ID NO:1 and 2 correspond to the VH of the 3.F2 antibody, SEQ ID NO:3 and 4 correspond to the VL of the 3.F2 antibody, SEQ ID NO:5 and 6 correspond to the VH of the 1.D2 antibody, SEQ ID NO:7 and 8 correspond to the VL of the 1.D2 antibody, SEQ ID NO:9 and 10 correspond to the VH of the 2.E8 antibody, and SEQ ID NO:11 and 12 correspond to the VL of the 2.E8 antibody.

For nucleic acids, the term "substantial homology" indicates that two nucleic acids, or designated sequences thereof, when optimally aligned and compared, are identical, with appropriate nucleotide insertions or deletions, in at least about 80% of the nucleotides, usually at least about 90% to 95%, and more preferably at least about 98% to 99.5% of the nucleotides. Alternatively, substantial homology exists when the segments will hybridize under selective hybridization conditions, to the complement of the strand.

The percent identity between two sequences is a function of the number of identical positions shared by the sequences (i.e., % homology = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. The percent identity between two nucleotide or amino acid sequences can also determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. (48):444-453 (1970)) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The sequences of the present invention can further be used as a "query sequence" to perform a search against public databases to, for example, identify related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, word length = 12 to obtain nucleotide sequences homologous to the nucleic acid molecules of the invention. BLAST protein searches can be performed with the XBLAST program, score = 50, wordlength = 3 to obtain amino acid sequences homologous to the protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used. See http://www.ncbi.nlm.nih.gov.

The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art. See, F. Ausubel, et al., ed. Current Protocols in Molecular Biology, Greene Publishing and Wiley Interscience, New York (1987).

Nucleic acid compositions, while often in a native sequence (except for modified restriction sites and the like), from either cDNA, genomic or mixtures may be mutated, thereof in accordance with standard techniques to provide gene sequences. For coding sequences, these mutations, may affect amino acid sequence as desired. In particular, DNA sequences substantially homologous to or derived from native V, D, J, constant, switches and other such sequences described herein are contemplated (where "derived" indicates that a sequence is identical or modified from another sequence).

A nucleic acid is "operably linked" when it is placed into a functional relationship with another nucleic acid sequence. For instance, a promoter or enhancer is operably linked to a coding sequence if it affects the transcription of the sequence. With respect to transcription regulatory sequences, operably linked means that the DNA sequences being linked are contiguous and, where necessary to join two protein coding regions, contiguous and in reading frame. For switch sequences, operably linked indicates that the sequences are capable of effecting switch recombination.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

Various aspects of the invention are described in further detail in the following subsections.

### I. Production of Human Antibodies to HER2/neu

The monoclonal antibodies (mAbs) of the invention can be produced by a variety of techniques, including conventional monoclonal antibody methodology e.g., the standard somatic cell hybridization technique of Kohler and Milstein, Nature 256: 495 (1975). Although somatic cell hybridization procedures are preferred, in principle, other techniques for producing monoclonal antibody can be employed e.g., viral or oncogenic transformation of B lymphocytes.

The preferred animal system for preparing hybridomas is the murine system. Hybridoma production in the mouse is a very well-established procedure. Immunization protocols and techniques for isolation of immunized splenocytes for fusion are known in the art. Fusion partners (e.g., murine myeloma cells) and fusion procedures are also known.

In a preferred embodiment, human monoclonal antibodies directed against HER2/neu can be generated using transgenic mice carrying the parts of the human immune system rather than the mouse system. These transgenic mice, referred to herein as "HuMAb" mice, contain a human immunoblobulin gene miniloci that encodes unrearranged human heavy (µ and γ) and κ light chain immunoglobulin sequences, together with targeted mutations that inactivate the endogenous µ and κ chain loci (Lonberg, N. et al. (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg, N. *et al.* (1994), *supra;* reviewed in Lonberg, N. (1994) Handbook of Experimental Pharmacology 113:49-101; Lonberg, N. and Huszar, D. (1995) Intern. Rev. Immunol. Vol. 13: 65-93, and Harding, F. and Lonberg, N. (1995) Ann. N. Y. Acad. Sci 764:536-546). The preparation of HuMab mice is described in detail Section II below and in Taylor, L. et al. (1992) Nucleic Acids Research 20:6287-6295; Chen, J. et al. (1993) International Immunology 5: 647-656; Tuaillon et al. (1993) Proc. Natl. Acad. Sci USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Lonberg et al., (1994) Nature 368(6474): 856-859; Lonberg, N. (1994) Handbook of Experimental Pharmacology 113:49-101; Taylor, L. et al. (1994) International Immunology 6: 579-591; Lonberg, N. and Huszar, D. (1995) Intern. Rev. Immunol. Vol. 13: 65-93; Harding, F. and Lonberg, N. (1995) Ann. N.Y. Acad Sci 764:536-546; Fishwild, D. et al. (1996) Nature Biotechnology 14: 845-851. See further, U.S. Patent Nos. 5,545,806; 5,569,825; 5,625,126; 5,633,425; 5,789,650; 5,877,397; 5,661,016; 5,814,318; 5,874,299; and 5,770,429; all to Lonberg and Kay, and GenPharm International; U.S. Patent No. 5,545,807 to Surani et al.*;* International Publication Nos. WO 98/24884, published on June 11, 1998; WO 94/25585, published November 10, 1994; WO 93/1227, published June 24, 1993; WO 92/22645, published December 23, 1992; WO 92/03918, published March 19, 1992. Alternatively, the HCO12 transgenic mice described in Example 2, can be used to generate human anti-HER2/neu antibodies.

### HuMab Immunizations

To generate fully human monoclonal antibodies to HER2/neu, HuMab mice can be immunized with a purified or enriched preparation of the HER2/neu antigen and/or cells expressing HER2/neu, as described by Lonberg, N. et al. (1994) Nature 368(6474): 856-859; Fishwild, D. et al. (1996) Nature Biotechnology 14: 845-851 and WO 98/24884. Preferably, the mice will be 6-16 weeks of age upon the first infusion. For example, a purified or enriched preparation (5-20 µg) of the HER2/neu antigen can be used to immunize the HuMab mice intraperitoneally. In the event that immunizations using a purified or enriched preparation of the HER2/neu antigen do not result in antibodies, mice can also be immunized with cells expressing HER2/neu, e.g., a tumor cell line, to promote immune responses.

Cumulative experience with various antigens has shown that the HuMAb transgenic mice respond best when initially immunized intraperitoneally (IP) with antigen in complete Freund's adjuvant, followed by every other week IP immunizations (up to a total of 6) with antigen in incomplete Freund's adjuvant. The immune response can be monitored over the course of the immunization protocol with plasma samples being obtained by retroorbital bleeds. The plasma can be screened by ELISA (as described below), and mice with sufficient titers of anti-HER2/neu human immunoglobulin can be used for fusions. Mice can be boosted intravenously with antigen 3 days before sacrifice and removal of the spleen. It is expected that 2-3 fusions for each antigen may need to be performed. Six mice will be immunized for each antigen. For example, a total of twelve HuMAb mice of the HC07 and HC012 strains can be immunized.

### Generation of Hybridomas Producing Human Monoclonal Antibodies to HER2/neu

The mouse splenocytes can be isolated and fused with PEG to a mouse myeloma cell line based upon standard protocols (8, 13). The resulting hybridomas are then screened for the production of antigen-specific antibodies. For example, single cell suspensions of splenic lymphocytes from immunized mice are fused to one-sixth the number of P3X63-Ag8.653 nonsecreting mouse myeloma cells (ATCC, CRL 1580) with 50% PEG. Cells are plated at approximately 2 x 10⁵ in flat bottom microtiter plate, followed by a two week incubation in selective medium containing 20% fetal Clone Serum, 18% "653" conditioned media, 5% origen (IGEN), 4 mM L-glutamine, 1 mM L~glutamine, 1 mM sodium pyruvate, 5mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After two weeks, cells are cultured in medium in which the HAT is replaced with HT. Individual wells are then screened by ELISA for human anti-HER2/neu monoclonal IgM and IgG antibodies. Once extensive hybridoma growth occurs, medium is observed usually after 10-14 days. The antibody secreting hybridomas are replated, screened again, and if still positive for human IgG, anti-HER2/neu monoclonal antibodies, can be subcloned at least twice by limiting dilution. The stable subclones are then cultured *in vitro* to generate small amounts of antibody in tissue culture medium for characterization.

### Characterization of Binding of Human Monoclonal Antibodies to HER2/neu

To characterize binding of human monoclonal HER2/neu antibodies of the invention, sera from immunized mice can be tested, for example, by ELISA. Briefly, microtiter plates are coated with purified HER2/neu at 0.25 µg/ml in PBS, and then blocked with 5% bovine serum albumin (BSA) in PBS. Dilutions of plasma from HER2/neu-immunized mice are added to each well and incubated for 1-2 hours at 37°C. The plates are washed with PBS/Tween and then incubated with a goat-anti-human IgG Fc-specific polyclonal reagent conjugated to alkaline phosphatase for 1 hour at 37°C. After washing, the plates are developed with pNPP substrate (1 mg/ml), and analyzed at OD of 405-650. Preferably, mice which develop the highest titers will be used for fusions.

Alternatively, microtiter plates are coated with mouse anti-HER2/neu antibody, blocked in 5% BSA, and incubated with the culture supernatant from overgrown cells expressing HER2/neu, e.g., SKBR-3 tumor cells, thus capturing HER2/neu antigen shed by the cells. Dilutions of purified human anti-HER2/neu antibodies are added to each well, incubated, and binding detected as described above.

An ELISA assay as described above can also be used to screen for antibodies and, thus, hybridomas that produce antibodies that show positive reactivity with the HER2/neu immunogen. Hybridomas that bind with high avidity to HER2/neu will be subcloned and further characterized. One clone from each hybridoma, which retains the reactivity of the parent cells (by ELISA), can be chosen for making a 5-10 vial cell bank stored at -140 °C, and for antibody purification.

To purify human anti-HER2/neu antibodies, selected hybridomas can be grown in two-liter spinner-flasks for monoclonal antibody purification. Supernatants can be filtered and concentrated before affinity chromatography with protein A-sepharose (Pharmacia, Piscataway, NJ). Eluted IgG can be checked by gel electrophoresis and high performance liquid chromatography to ensure purity. The buffer solution can be exchanged into PBS, and the concentration can be determined by OD₂₈₀ using 1.43 extinction coefficient. The monoclonal antibodies can be aliquoted and stored at -80 °C.

To determine if the selected human anti-HER2/neu monoclonal antibodies bind to unique epitopes, each antibody can be biotinylated using commercially available reagents (Pierce, Rockford, IL). Competition studies using unlabeled monoclonal antibodies and biotinylated monoclonal antibodies can be performed using HER2/neu coated-ELISA plates as described above. Biotinylated mAb binding can be detected with a strep-avidin-alkaline phosphatase probe.

To determine the isotype of purified antibodies, isotype ELISAs can be performed. Wells of microtiter plates can be coated with 10 µg/ml of anti-human Ig overnight at 4°C. After blocking with 5% BSA, the plates are reacted with 10 µg/ml of monoclonal antibodies or purified isotype controls, at ambient temperature for two hours. The wells can then be reacted with either human IgGI or human IgM-specific alkaline phosphatase-conjugated probes. Plates are developed and analyzed as described above.

In order to demonstrate binding of monoclonal antibodies to live cells expressing the HER2/neu receptor, flow cytometry can be used. Briefly, cell lines expressing HER2/neu (grown under standard growth conditions) are mixed with various concentrations of monoclonal antibodies in PBS containing 0.1% Tween 80 and 20% mouse serum, and incubated at 37°C for 1 hour. After washing, the cells are reacted with Fluorescein-labeled anti-human IgG antibody under the same conditions as the primary antibody staining. The samples can be analyzed by FACScan instrument using light and side scatter properties to gate on single cells. An alternative assay using fluorescence microscopy may be used (in addition to or instead of) the flow cytometry assay. Cells can be stained exactly as described above and examined by fluorescence microscopy. This method allows visualization of individual cells, but may have diminished sensitivity depending on the density of the antigen.

Anti-HER2/neu human IgGs can be further tested for reactivity with the HER2/neu antigen by Western blotting. Briefly, cell extracts from cells expressing HER2/neu can be prepared and subjected to sodium dodecyl sulfate polyacrylamide gel electrophoresis. After electrophoresis, the separated antigens will be transferred to nitrocellulose membranes, blocked with 20% mouse serum, and probed with the monoclonal antibodies to be tested. Human IgG binding can be detected using anti-human IgG alkaline phophatase and developed with BCIP/NBT substrate tablets (Sigma Chem. Co., St. Louis, MO).

### Phagocytic and Cell Killing Activities of Human Monoclonal Antibodies to HER2/neu

In addition to binding specifically to HER2/neu, human monoclonal anti-HER2/neu antibodies can be tested for their ability to mediate phagocytosis and killing of cells expressing HER2/neu. The testing of monoclonal antibody activity *in vitro* will provide an initial screening prior to testing *in vivo* models. Briefly, polymorphonuclear cells (PMN), or other effector cells (e.g., IFN-γ induced marcophages), from healthy donors can be purified by Ficoll Hypaque density centrifugation, followed by lysis of contaminating erythrocytes. Washed PMNs, can be suspended in RPMI supplemented with 10% heat-inactivated fetal calf serum and mixed with ⁵¹Cr labeled cells expressing HER2/neu, at various ratios of effector cells to tumor cells (E:T). Purified human anti-HER2/neu IgGs can then be added at various concentrations. Irrelevant human IgG can be used as negative control. Assays can be carried out for 4-16 hours at 37°C. Samples can be assayed for cytolysis by measuring ⁵¹Cr release into the culture supernatant. Anti-HER2/neu monoclonal can also be tested in combinations with each other to determine whether cytolysis is enhanced with multiple monoclonal antibodies.

Human monoclonal antibodies which bind to HER2/neu also can be tested in an *in vivo* model (e.g., in mice) to determine their efficacy in mediating phagocytosis and killing of cells expressing HER2/neu, e.g., tumor cells. These antibodies can be selected, for example, based on the following criteria, which are not intended to be exclusive:
**1.)** binding to live cells expressing HER2/neu;
**2.)** high affinity of binding to HER2/neu;
**3.)** binding to a unique epitope on HER2/neu (to eliminate the possibility that monoclonal antibodies with complimentary activities when used in combination would compete for binding to the same epitope);
**4.)** opsonization of cells expressing HER2/neu;
**5.)** mediation of growth inhibition, phagocytosis and/or killing of cells expressing HER2/neu in the presence of human effector cells;
**6.)** internalization by HER2/neu expressing cells after binding to HER2/neu.

Preferred human monoclonal antibodies of the invention meet one or more, and preferably all, of these criteria. In a particular embodiment, the human monoclonal antibodies are used in combination, e.g., as a pharmaceutial composition comprising two or more anti-HER2/neu monoclonal antibodies or fragments thereof. For example, human anti-HER2/neu monoclonal antibodies having different, but complementary activities can be combined in a single therapy to achieve a desired therapeutic or diagnostic effect. An illustration of this would be a composition containing an anti-HER2/neu human monoclonal antibody that mediates highly effective killing of target cells in the presence of effector cells, combined with another human anti-HER2/neu monoclonal antibody that inhibits the growth of cells expressing HER2/neu.

### II. Production of Transgenic Nonhuman Animals Which Generate Human Monoclonal Anti-HER2/neu Antibodies

Transgenic non-human animals can be provided, e.g. transgenic mice, which arc capable of expressing human monoclonal antibodies that specifically bind to HER2/neu, preferably with high affinity. In a preferred embodiment, the transgenic non-human animals, e.g., the transgenic mice (HuMab mice), have a genome comprising a human heavy chain transgene and a light chain transgene. In one embodiment, the transgenic non-human animals, e.g., the transgenic mice, have been immunized with a purified or enriched preparation of HER2/neu antigen and/or cells expressing HER2/neu. Preferably, the transgenic non-human animals, e.g., the transgenic mice, are capable of producing multiple isotypes of human monoclonal antibodies to HER2/neu (e.g., IgG, IgA and/or IgE) by undergoing V-D-J recombination and isotype switching. Isotype switching may occur by, e.g., classical or non-classical isotype switching.

The design of a transgenic non-human animal that responds to foreign antigen stimulation with a heterologous antibody repertoire, requires that the heterologous immunoglobulin transgenes contain within the transgenic animal function correctly throughout the pathway of B-cell development. In a preferred embodiment, correct function of a heterologous heavy chain transgene includes isotype switching. Accordingly, the transgenes of the invention are constructed so as to produce isotype switching and one or more of the following: (1) high level and cell-type specific expression, (2) functional gene rearrangement, (3) activation of and response to allelic exclusion, (4) expression of a sufficient primary repertoire, (5) signal transduction, (6) somatic hypermutation, and (7) domination of the transgene antibody locus during the immune response.

Not all of the foregoing criteria need be met. For example, in those embodiments wherein the endogenous immunoglobulin loci of the transgenic animal are functionally disrupted, the transgene need not activate allelic exclusion. Further, in those embodiments wherein the transgene comprises a functionally rearranged heavy and/or light chain immunoglobulin gene, the second criteria of functional gene rearrangement is unnecessary, at least for that transgene which is already rearranged. For background on molecular immunology, see, Fundamental Immunology, 2nd edition (1989), Paul William E., ed. Raven Press, N.Y.

In certain embodiments, the transgenic non-human animals used to generate the human monoclonal antibodies of the invention contain rearranged, unrearranged or a combination of rearranged and unrearranged heterologous immunoglobulin heavy and light chain transgenes in the germline of the transgenic animal. Each of the heavy chain transgenes comprises at least one C_{H} gene. In addition, the heavy chain transgene may contain functional isotype switch sequences, which are capable of supporting isotype switching of a heterologous transgene encoding multiple C_{H} genes in the B-cells of the transgenic animal. Such switch sequences may be those which occur naturally in the germline immunoglobulin locus from the species that serves as the source of the transgene C_{H} genes, or such switch sequences may be derived from those which occur in the species that is to receive the transgene construct (the transgenic animal). For example, a human transgene construct that is used to produce a transgenic mouse may produce a higher frequency of isotype switching events if it incorporates switch sequences similar to those that occur naturally in the mouse heavy chain locus, as presumably the mouse switch sequences are optimized to function with the mouse switch recombinase enzyme system, whereas the human switch sequences are not. Switch sequences may be isolated and cloned by conventional cloning methods, or may be synthesized *de novo* from overlapping synthetic oligonucleotides designed on the basis of published sequence information relating to immunoglobulin switch region sequences (Mills et al., Nucl. Acids Res. 15:7305-7316 (1991); Sideras et al., Intl. Immunol. 1:631-642. (1989).
For each of the foregoing transgenic animals, functionally rearranged heterologous heavy and light chain immunoglobulin transgenes are found in a significant fraction of the B-cells of the transgenic animal (at least 10 percent).

The transgenes used to generate the transgenic animals of the invention include a heavy chain transgene comprising DNA encoding at least one variable gene segment, one diversity gene segment, one joining gene segment and at least one constant region gene segment. The immunoglobulin light chain transgene comprises DNA encoding at least one variable gene segment, one joining gene segment and at least one constant region gene segment. The gene segments encoding the light and heavy chain gene segments are heterologous to the transgenic non-human animal in that they are derived from, or correspond to, DNA encoding immunoglobulin heavy and light chain gene segments from a species not consisting of the transgenic non-human animal. In one aspect of the invention, the transgene is constructed such that the individual gene segments are unrearranged, i.e., not rearranged so as to encode a functional immunoglobulin light or heavy chain. Such unrearranged transgenes support recombination of the V, D, and J gene segments (functional rearrangement) and preferably support incorporation of all or a portion of a D region gene segment in the resultant rearranged immunoglobulin heavy chain within the transgenic non-human animal when exposed to the HER2/neu antigen.

In an alternate embodiment, the transgenes comprise an unrearranged "mini-locus". Such transgenes typically comprise a substantial portion of the C, D, and J segments as well as a subset of the V gene segments. In such transgene constructs, the various regulatory sequences, e.g. promoters, enhancers, class switch regions, splice-donor and splice-acceptor sequences for RNA processing, recombination signals and the like, comprise corresponding sequences derived from the heterologous DNA. Such regulatory sequences may be incorporated into the transgene from the same or a related species of the non-human animal used in the invention. For example, human immunoglobulin gene segments may be combined in a transgene with a rodent immunoglobulin enhancer sequence for use in a transgenic mouse. Alternatively, synthetic regulatory sequences may be incorporated into the transgene, wherein such synthetic regulatory sequences are not homologous to a functional DNA sequence that is known to occur naturally in the genomes of mammals. Synthetic regulatory sequences are designed according to consensus rules, such as, for example, those specifying the permissible sequences of a splice-acceptor site or a promoter/enhancer motif. For example, a minilocus comprises a portion of the genomic immunoglobulin locus having at least one internal (i.e., not at a terminus of the portion) deletion of a non-essential DNA portion (e.g., intervening sequence; intron or portion thereof) as compared to the naturally-occurring germline 1g locus.

In a preferred embodiment of the invention, the transgenic animal used to generate human antibodies to HER2/neu contains at least one, typically 2-10, and sometimes 25-50 or more copies of the transgene described in Example 12 of WO 98/24884 (e.g., pHC1 or pHC2) bred with an animal containing a single copy of a light chain transgene described in Examples 5, 6, 8, or 14 of WO 98/24884, and the offspring bred with the J_{H} deleted animal described in Example 10 of WO 98/24884

Animals are bred to homozygosity for each of these three traits. Such animals have the following genotype: a single copy (per haploid set of chromosomes) of a human heavy chain unrearranged mini-locus (described in Example 12 of WO 98/24884), a single copy (per haploid set of chromosomes) of a rearranged human K light chain construct (described in Example 14 of WO 98/24884), and a deletion at each endogenous mouse heavy chain locus that removes all of the functional J_{H} segments (described in Example 10 of WO 98/24884). Such animals are bred with mice that are homozygous for the deletion of the J_{H} segments (Examples 10 of WO 98/24884) to produce offspring that are homozygous for the J_{H} deletion and hemizygous for the human heavy and light chain constructs. The resultant animals are injected with antigens and used for production of human monoclonal antibodies against these antigens.

B cells isolated from such an animal are monospecific with regard to the human heavy and light chains because they contain only a single copy of each gene. Furthermore, they will be monospecific with regards to human or mouse heavy chains because both endogenous mouse heavy chain gene copies are nonfunctional by virtue of the deletion spanning the J_{H} region introduced as described in Example 9 and 12 of WO 98/24884. Furthermore, a substantial fraction of the B cells will be monospecific with regards to the human or mouse light chains because expression of the single copy of the rearranged human κ light chain gene will allelically and isotypically exclude the rearrangement of the endogenous mouse κ and lambda chain genes In a significant fraction of B-cells.

The transgenic mouse of the preferred embodiment will exhibit immunoglobulin production with a significant repertoire, ideally substantially similar to that of a native mouse. Thus, for example, in embodiments where the endogenous Ig genes have been inactivated, the total immunoglobulin levels will range from about 0.1 to 10 mg/ml of serum, preferably 0.5 to 5 mg/ml, ideally at least about 1.0 mg/ml. When a transgene capable of effecting a switch to IgG from IgM has been introduced into the transgenic mouse, the adult mouse ratio of serum IgG to IgM is preferably about 10:1. The IgG to IgM ratio will be much lower in the immature mouse. In general, greater than about 10%, preferably 40 to 80% of the spleen and lymph node B cells express exclusively human IgG protein.

The repertoire will ideally approximate that shown in a non-transgenic mouse, usually at least about 10% as high, preferably 25 to 50% or more. Generally, at least about a thousand different immunoglobulins (ideally IgG), preferably 10⁴ to 10⁶ or more, will be produced, depending primarily on the number of different V, J and D regions introduced into the mouse genome. These immunoglobulins will typically recognize about one-half or more of highly antigenic proteins, e.g., staphylococcus protein A. Typically, the immunoglobulins will exhibit an affinity for preselected antigens of at least about 10⁷M⁻¹, preferably at least about 10⁹M⁻¹, more preferably at least about 10¹⁰M⁻¹, 10¹¹M⁻¹, 10¹²M⁻¹ or greater, e.g., up to 10¹³M⁻¹ or greater.

In some embodiments, it may be preferable to generate mice with predetermined repertoires to limit the selection of V genes represented in the antibody response to a predetermined antigen type. A heavy chain transgene having a predetermined repertoire may comprise, for example, human VH genes which are preferentially used in antibody responses to the predetermined antigen type in humans. Alternatively, some VH genes may be excluded from a defined repertoire for various reasons (e.g., have a low likelihood of encoding high affinity V regions for the predetermined antigen; have a low propensity to undergo somatic mutation and affinity sharpening; or are immunogenic to certain humans). Thus, prior to rearrangement of a transgene containing various heavy or light chain gene segments, such gene segments may be readily identified, e.g. by hybridization or DNA sequencing, as being from a species of organism other than the transgenic animal.

The transgenic mice of the present invention can be immunized with a purified or enriched preparation of HER2/neu antigen and/or cells expressing HER2/neu as described previously. The mice will produce B cells which undergo class-switching via intratransgene switch recombination (cis-switching) and express immunoglobulins reactive with HER2/neu. The immunoglobulins can be human sequence antibodies, wherein the heavy and light chain polypeptides are encoded by human transgene sequences, which may include sequences derived by somatic mutation and V region recombinatorial joints, as well as germline-encoded sequences; these human sequence immunoglobulins can be referred to as being substantially identical to a polypeptide sequence encoded by a human V_{L} or V_{H} gene segment and a human J_{L} or J_{L} segment, even though other non-germline sequences may be present as a result of somatic mutation and differential V-J and V-D-J recombination joints. With respect to such human sequence antibodies, the variable regions of each chain are typically at least 80 percent encoded by human germline V, J, and, in the case of heavy chains, D, gene segments; frequently at least 85 percent of the variable regions are encoded by human germline sequences present on the transgene; often 90 or 95 percent or more of the variable region sequences are encoded by human germline sequences present on the transgene. However, since non-germline sequences are introduced by somatic mutation and VJ and VDJ joining, the human sequence antibodies will frequently have some variable region sequences (and less frequently constant region sequences) which are not encoded by human V, D, or J gene segments as found in the human transgene(s) in the germline of the mice. Typically, such non-germline sequences (or individual nucleotide positions) will cluster in or near CDRs, or in regions where somatic mutations are known to cluster.

The human sequence antibodies which bind to the predetermined antigen can result from isotype switching, such that human antibodies comprising a human sequence γ chain (such as γ1, γ2a, γ2B, or γ3) and a human sequence light chain (such as K) are produced. Such isotype-switched human sequence antibodies often contain one or more somatic mutation(s), typically in the variable region and often in or within about 10 residues of a CDR) as a result of affinity maturation and selection of B cells by antigen, particularly subsequent to secondary (or subsequent) antigen challenge. These high affinity human sequence antibodies may have binding affinities of at least 1 x 10⁹ M⁻¹, typically at least 5 x 10⁹ M⁻¹, frequently more than 1 x 10¹⁰ M⁻¹, and sometimes 5 x 10¹⁰ M⁻¹ to 1 x 10¹¹ M⁻¹ or greater.

Another aspect of the invention pertains to the B cells from such mice which can be used to generate hybridomas expressing human monoclonal antibodies which bind with high affinity (e.g., greater than 2 x 10⁹ M⁻¹) to HER2/neu. Thus, in another embodiment of the invention, these hybridomas are used to generate a composition comprising an immunoglobulin having an affinity constant (Ka) of at least 2 x 10⁹ M⁻¹ for binding HER2/neu, wherein said immunoglobulin comprises:
a human sequence light chain composed of (1) a light chain variable region having a polypeptide sequence which is substantially identical to a polypeptide sequence encoded by a human V_{L} gene segment and a human J_{L} segment, and (2) a light chain constant region having a polypeptide sequence which is substantially identical to a polypeptide sequence encoded by a human C_{L} gene segment; and
a human sequence heavy chain composed of a (1) a heavy chain variable region having a polypeptide sequence which is substantially identical to a polypeptide sequence encoded by a human V_{H} gene segment, optionally a D region, and a human J_{H} segment, and (2) a constant region having a polypeptide sequence which is substantially identical to a polypeptide sequence encoded by a human C_{H} gene segment.

The development of high affinity human monoclonal antibodies against HER2/neu is facilitated by a method for expanding the repertoire of human variable region gene segments in a transgenic mouse having a genome comprising an integrated human immunoglobulin transgene, said method comprising introducing into the genome a V gene transgene comprising V region gene segments which arc not present in said integrated human immunoglobulin transgene. Often, the V region transgene is a yeast artificial chromosome comprising a portion of a human V_{H} or V_{L} (V_{K}) gene segment array, as may naturally occur in a human genome or as may be spliced together separately by recombinant methods, which may include out-of-order or omitted V gene segments. Often at least five or more functional V gene segments are contained on the YAC. In this variation, it is possible to make a transgenic mouse produced by the V repertoire expansion method, wherein the mouse expresses an immunoglobulin chain comprising a variable region sequence encoded by a V region gene segment present on the V region transgene and a C region encoded on the human Ig transgene. By means of the V repertoire expansion method, transgenic mice having at least 5 distinct V genes can be generated; as can mice containing at least about 24 V genes or more. Some V gene segments may be non-functional (e.g., pseudogenes and the like); these segments may be retained or may be selectively deleted by recombinant methods available to the skilled artisan, if desired.

Once the mouse germline has been engineered to contain a functional YAC having an expanded V segment repertoire, substantially not present in the human 1g transgene containing the J and C gene segments, the trait can be propagated and bred into other genetic backgrounds, including backgrounds where the functional YAC having an expanded V segment repertoire is bred into a mouse germline having a different human Ig transgene. Multiple functional YACs having an expanded V segment repertoire may be bred into a germline to work with a human Ig transgene (or multiple human Ig transgenes). Although referred to herein as YAC transgenes, such transgenes when integrated into the genome may substantially lack yeast sequences, such as sequences required for autonomous replication in yeast; such sequences may optionally be removed by genetic engineering (e.g., restriction digestion and pulsed-field gel electrophoresis or other suitable method) after replication in yeast is no longer necessary (i.e., prior to introduction into a mouse ES cell or mouse prozygote). Methods of propagating the trait of human sequence immunoglobulin expression, include breeding a transgenic mouse having the human Ig transgene(s), and optionally also having a functional YAC having an expanded V segment repertoire. Both V_{H} and V_{L} gene segments may be present on the YAC. The transgenic mouse may be bred into any background desired by the practitioner, including backgrounds harboring other human transgenes, including human Ig transgenes and/or transgenes encoding other human lymphocyte proteins. The invention also provides a high affinity human sequence immunoglobulin produced by a transgenic mouse having an expanded V region repertoire YAC transgene. Although the foregoing describes a preferred embodiment of the transgenic animal, other embodiments are contemplated which have been classified in four categories:
I. Transgenic animals containing an unrearranged heavy and rearranged light immunoglobulin transgene;
II. Transgenic animals containing an unrearranged heavy and unrearranged light immunoglobulin transgene;
III. Transgenic animal containing rearranged heavy and an unrearranged light immunoglobulin transgene; and
IV. Transgenic animals containing rearranged heavy and rearranged light immunoglobulin transgenes.

Of these categories of transgenic animal, the preferred order of preference is as follows II > I > III > IV where the endogenous light chain genes (or at least the K gene) have been knocked out by homologous recombination (or other method) and I > II > III >IV where the endogenous light chain genes have not been knocked out and must be dominated by allelic exclusion.

### III. Bispecific/ Multispecific Molecules Which Bind to HER2/neu

In yet another embodiment of the invention, human monoclonal antibodies to HER2/neu (or antigen-binding portions thereof, or single chain antibodies thereof) are derivatized or linked to another functional molecule, such as another antibody or antibody fragment (including single chain antibodies and Fab' antibody fragments) or a protein ligand, to generate a bispecific or multispecific molecule which binds to multiple binding sites or target epitopes.

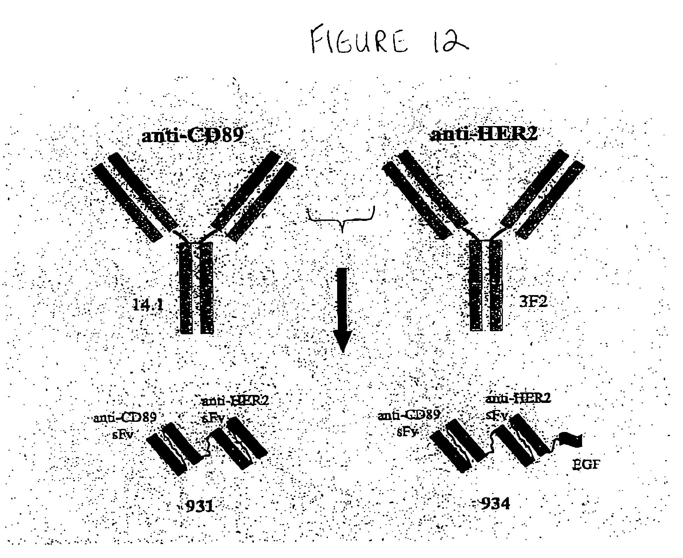

In a particular embodiment, the invention provides a bispecific molecule that includes a first portion comprising a human monoclonal anti-HER2/neu antibody (or antigen-binding portion thereof, or single chain antibody thereof) functionally linked (e.g., by chemical coupling, genetic fusion, noncovalent association or otherwise) to a second antibody (or antigen-binding portion thereof, or single chain antibody thereof) which binds to an Fc receptor, typically FcδRI or FcαR (Figure 12). The invention further provides multispecific molecules which further include a third portion which binds to a tumor cell antigen other than HER2/neu, such as EGF-R. In a particular embodiment, the third portion is EGF. Thus, particular multispecific molecules of the invention contain a human anti-HER2/neu antibody (or antigen-binding portion thereof, or single chain antibody thereof) linked to an anti-FcR antibody (or antigen-binding portion thereof, or single chain antibody thereof) linked to EGF (Figure 12).

Accordingly, the present invention includes bispecific and multispecific molecules comprising at least one first binding specificity for HER2/neu and a second binding specificity for a second target epitope. In a particular embodiment of the invention, the second target epitope is an Fc receptor, e.g., human FcγRI or a human Fcα receptor. Therefore, the invention includes bispecific and multispecific molecules capable of binding both to FcγR, FcαR or FcεR expressing effector cells (e.g., monocytes, macrophages or polymorphonuclear cells (PMNs)), and to target cells expressing HER2/neu. These bispecific and multispecific molecules target HER2/neu expressing cells to effector cells, and trigger Fc receptor-mediated effector cell activities, such as phagocytosis of a the HER2/neu expressing cells, antibody dependent cell-mediated cytotoxicity (ADCC), cytokine release, or generation of superoxide anion.

Bispecific and multispecific molecules of the invention can further include a third binding specificity for a target other than HER2/neu, also referred to as an "anti-enhancement factor (EF)", in addition to an anti-Fc binding specificity and an anti-HER2/neu binding specificity. For example, the third binding specificity can bind to a cell surface protein involved in cytotoxic activity and thereby increases the immune response against the target cell. The third binding specificity can be an antibody (including ScFv), functional antibody fragment or a ligand that binds to a given molecule, e.g., an antigen or a receptor, and thereby results in an enhancement of the effect of the binding determinants for the F_{c} receptor or target cell antigen. The third third binding specificity or "anti-enhancement factor portion" also can bind an F_{c} receptor or a target cell antigen. Alternatively, the anti-enhancement factor portion can bind to an entity that is different from the entity to which the first and second binding specificities bind. For example, the anti-enhancement factor portion can bind a cytotoxic T-cell (e.g. via CD2, CD3, CD8, CD28, CD4, CD40, ICAM-1 or other immune cell that results in an increased immune response against the target cell).

In one embodiment, the bispecific and multispecific molecules of the invention comprise as a binding specificity at least one antibody, or an antibody fragment thereof, including, e.g., an Fab, Fab', F(ab')₂, Fv, or a single chain Fv. The antibody may also be a light chain or heavy chain dimer, or any minimal fragment thereof such as a Fv or a single chain construct as described in Ladner et al. U.S. Patent No. 4,946,778, issued August 7, 1990.

In another embodiment, bispecific and multispecific molecules of the invention comprise (a) a human monoclonal antibody against HER2/ncu (e.g., antibody 3.F2 described herein), or an antibody fragment thereof including, e.g., an Fab, Fab', F(ab')₂, Fv, or a single chain Fv, and (b) a human monoclonal antibody against FcγR or an FcαR (e.g., antibody 14.1 described herein) present on the surface of an effector cell, or an antibody fragment thereof, including, e.g., an Fab, Fab', F(ab')₂, Fv, or a single chain Fv. Preferably, the human anti-Fc antibody is not blocked by human IgG or IgA when binding to effector cells.

As used herein, the term "IgG receptor" refers to any of the eight γ-chain genes located on chromosome 1. These genes encode a total of twelve transmembrane or soluble receptor isoforms which arc grouped into three Fcγ receptor classes: FcγRI (CD64), FcγRII(CD32), and FcγRIII (CD16). In one preferred embodiment, the Fcγ receptor a human high affinity FcγRI. The human FcγRI is a 72 kDa molecule, which shows high affinity for monomeric IgG (10⁸ - 10⁹ M⁻¹).

The production and characterization of these preferred monoclonal antibodies are described by Fanger et al. in PCT application WO 88/00052 and in U.S. Patent No. 4,954,617. These antibodies bind to an epitope of FcγRI, FcγRII or FcγRIII at a site which is distinct from the Fcγ binding site of the receptor and, thus, their binding is not blocked substantially by physiological levels of IgG. Specific anti-FcγRI antibodies useful in this invention are mAb 22, mAb 32, mAb 44, mAb 62 and mAb 197. The hybridoma producing mAb 32 is available from the American Type Culture Collection, ATCC Accession No. HB9469. Anti-FcγRI mAb 22, F(ab')₂ fragments of mAb 22, and can be obtained from Medarex, Inc. (Annandale, N.J.). In other embodiments, the anti-Fcγ receptor antibody is a humanized form of monoclonal antibody 22 (H22). The production and characterization of the H22 antibody is described in Graziano, R.F. et al. (1995) J. Immunol 155 (10): 4996-5002 and PCT/US93/10384. The H22 antibody producing cell line was deposited at the American Type Culture Collection on November 4, 1992 under the designation HA022CL1 and has the accession no. CRL 11177.

In still other preferred embodiments, the binding specificity for an Fc receptor is provided by an antibody that binds to a human IgA receptor, e.g., an Fc-alpha receptor (FcαR (CD89)). Preferably, the antibody binds to a human IgA receptor at a site that is not blocked by endogenous IgA. The term "IgA receptor" is intended to include the gene product of one α-gene (FcαRI) located on chromosome 19. This gene is known to encode several alternatively spliced transmembrane isoforms of 55 to 110 kDa. FcαRI (CD89) is constitutively expressed on monocytes/macrophages, eosinophilic and neutrophilic granulocytes, but not on non-effector cell populations. FcαRI has medium affinity (≈ 5 × 10⁷ M⁻¹) for both IgA1 and IgA2, which is increased upon exposure to cytokines such as G-CSF or GM-CSF (Morton, H.C. et al. (1996) Critical Reviews in Immunology 16:423-440). Four FcαRI-specific monoclonal antibodies, identified as A3, A59, A62 and A77, which bind FcαRI outside the IgA ligand binding domain, have been described (Monteiro, R.C. et al., 1992, J. Immunol. 148:1764). Also described herein is a fully human monoclonal antibody against FcαRI referred to as 14.1.

An "effector cell specific antibody" as used herein refers to an antibody or functional antibody fragment that binds the Fc receptor of effector cells. Preferred antibodies for use in the subject invention bind the Fc receptor of effector cells at a site which is not bound by endogenous immunoglobulin.

As used herein, the term "effector cell" refers to an immune cell which is involved in the effector phase of an immune response, as opposed to the cognitive and activation phases of an immune response. Exemplary immune cells include a cell of a myeloid or lymphoid origin, e.g., lymphocytes (e.g., B cells and T cells including cytolytic T cells (CTLs)), killer cells, natural killer cells, macrophages, monocytes, eosinophils, neutrophils, polymorphonuclear cells, granulocytes, mast cells, and basophils. Some effector cells express specific Fc receptors and carry out specific immune functions. In preferred embodiments, an effector cell is capable of inducing antibody-dependent cell-mediated cytotoxicity (ADCC), e.g., a neutrophil capable of inducing ADCC. For example, monocytes and macrophages which express FcR are involved in specific killing of target cells and presenting antigens to other components of the immune system, or binding to cells that present antigens. In other embodiments, an effector cell can phagocytose a target antigen, target cell, or microorganism. The expression of a particular FcR on an effector cell can be regulated by humoral factors such as cytokines. For example, expression of FcγRI has been found to be up-regulated by interferon gamma (IFN-γ). This enhanced expression increases the cytotoxic activity of FcγRI-bearing cells against targets. An effector cell can phagocytose or lyse a target antigen or a target cell.

"Target cell" shall mean any undesirable cell in a subject (e.g., a human or animal) that can be targeted by a composition (e.g., a human monoclonal antibody, a bispecific or a multispecific molecule) of the invention. In preferred embodiments, the target cell is a cell expressing, preferably overexpressing, HER2/neu. Cells expressing HER2/neu typically include tumor cells, including adenocarcinoma cells, e.g. salivary gland, stomach and kidney, a mammary gland carcinoma cells, lung carcinoma cells, squamous cell carcinoma cells, and ovarian cancer cells.

While human monoclonal antibodies are preferred, other antibodies which can be employed in the bispecific or multispecific molecules of the invention are murine, chimeric and humanized monoclonal antibodies.

Chimeric mouse-human monoclonal antibodies (i.e., chimeric antibodies) can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted. (see Robinson et al., International Patent Publication PCT/US86/02269; Akira, et al., European Patent Application 184,187; Taniguchi, M., European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. (1985) Nature 314:446-449; and Shaw et al., 1988, J. Natl Cancer Inst. 80:1553-1559).

The chimeric antibody can be further humanized by replacing sequences of the Fv variable region which are not directly involved in antigen binding with equivalent sequences from human Fv variable regions. General reviews of humanized chimeric antibodies are provided by Morrison, S. L., 1985, Science 229:1202-1207 and by Oi et al., 1986, BioTechniques 4:214. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from 7E3, an anti-GPII_{b}IIIₐ antibody producing hybridoma. The recombinant DNA encoding the chimeric antibody, or fragment thereof, can then be cloned into an appropriate expression vector. Suitable humanized antibodies can alternatively be produced by CDR substitution U.S. Patent 5,225,539; Jones et al. 1986 Nature 321:552-525; Verhoeyan et al. 1988 Science 239:1534; and Beidler et al. 1988 J. Immunol. 141:4053-4060.

All of the CDRs of a particular human antibody may be replaced with at least a portion of a non-human CDR or only some of the CDRs may be replaced with non-human CDRs. It is only necessary to replace the number of CDRs required for binding of the humanized antibody to the Fc receptor.

An antibody can be humanized by any method, which is capable of replacing at least a portion of a CDR of a human antibody with a CDR derived from a non-human antibody. Winter describes a method which may be used to prepare the humanized antibodies of the present invention (UK Patent Application GB 2188638A, filed on March 26, 1987). The human CDRs may be replaced with non-human CDRs using oligonucleotide site-directed mutagenesis as described in International Application WO 94/10332 entitled, *Humanized Antibodies to Fc Receptors for Immunoglobulin G on Human Mononuclear Phagocytes.*

Also within the scope of the invention are chimeric and humanized antibodies in which specific amino acids have been substituted, deleted or added. In particular, preferred humanized antibodies have amino acid substitutions in the framework region, such as to improve binding to the antigen. For example, in a humanized antibody having mouse CDRs, amino acids located in the human framework region can be replaced with the amino acids located at the corresponding positions in the mouse antibody. Such substitutions are known to improve binding of humanized antibodies to the antigen in some instances. Antibodies in which amino acids have been added, deleted, or substituted are referred to herein as modified antibodies or altered antibodies.

The term modified antibody is also intended to include antibodies, such as monoclonal antibodies, chimeric antibodies, and humanized antibodies which have been modified by, e.g., deleting, adding, or substituting portions of the antibody. For example, an antibody can be modified by deleting the constant region and replacing it with a constant region meant to increase half-life, e.g., serum half-life, stability or affinity of the antibody. Any modification is within the scope of the invention so long as the bispecific and multispecific molecule has at least one antigen binding region specific for an FcγR and triggers at least one effector function.

Bispecific and multispecific molecules of the present invention can be made using chemical techniques (see e.g., D. M. Kranz et al. (1981) Proc. Natl. Acad. Sci. USA 78:5807), "polydoma" techniques (See U.S. Patent 4,474,893, to Reading), or recombinant DNA techniques.

In particular, bispecific and multispecific molecules of the present invention can be prepared by conjugating the constituent binding specificities, e.g., the anti-FcR and anti-HER2/neu binding specificities, using methods known in the art and described in the examples provided herein. For example, each binding specificity of the bispecific and multispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetyl-thioacetate (SATA), N-succinimidyl-3-(2-pyridyldithio)propionate (SPDP), and sulfosuccinimidyl 4-(N-maleimidomethyl) cyclohaxane-1-carboxylate (sulfo-SMCC) (see e.g., Karpovsky et al. (1984) J. Exp. Med. 160:1686; Liu, MA et al. (1985) Proc. Natl. Acad. Sci. USA 82:8648). Other methods include those described by Paulus (Behring Ins. Mitt. (1985) No. 78, 118-132); Brennan et al. (Science (1985) 229:81-83), and Glennie et al. (J. Immunol. (1987) 139: 2367-2375). Preferred conjugating agents are SATA and sulfo-SMCC, both available from Pierce Chemical Co. (Rockford, IL).

When the binding specificities are antibodies (e.g., two humanized antibodies), they can be conjugated via sulfhydryl bonding of the C-terminus hinge regions of the two heavy chains. In a particularly preferred embodiment, the hinge region is modified to contain an odd number of sulfhydryl residues, preferably one, prior to conjugation.

Alternatively, both binding specificities can be encoded in the same vector and expressed and assembled in the same host cell. This method is particularly useful where the bispecific and multispecific molecule is a mAb x mAb, mAb x Fab, Fab x F(ab')₂ or ligand x Fab fusion protein. A bispecific and multispecific molecule of the invention, e.g., a bispecific molecule can be a single chain molecule, such as a single chain bispecific antibody, a single chain bispecific molecule comprising one single chain antibody and a binding determinant, or a single chain bispecific molecule comprising two binding determinants. Bispecific and multispecific molecules can also be single chain molecules or may comprise at least two single chain molecules. Methods for preparing bi- and multspecific molecules are described for example in U.S. Patent Number 5,260,203; U.S. Patent Number 5,455,030; U.S. Patent Number 4,881,175; U.S. Patent Number 5,132,405; U.S. Patent Number 5,091,513; U.S. Patent Number 5,476,786; U.S. Patent Number 5,013,653; U.S. Patent Number 5,258,498; and U.S. Patent Number 5,482,858.

Binding of the bispecific and multispecific molecules to their specific targets can be confirmed by enzyme-linked immunosorbent assay (ELISA), a radioimmunoassay (RIA), or a Western Blot Assay. Each of these assays generally detects the presence of protein-antibody complexes of particular interest by employing a labeled reagent (e.g., an antibody) specific for the complex of interest. For example, the FcR-antibody complexes can be detected using e.g., an enzyme-linked antibody or antibody fragment which recognizes and specifically binds to the antibody-FcR complexes. Alternatively, the complexes can be detected using any of a variety of other immunoassays. For example, the antibody can be radioactively labeled and used in a radioimmunoassay (RIA) (see, for example, Weintraub, B., Principles of Radioimmunoassays, Seventh Training Course on Radioligand Assay Techniques, The Endocrine Society, March, 1986. The radioactive isotope can be detected by such means as the use of a γ counter or a scintillation counter or by autoradiography.

### IV. Antibody Conjugates/Immunotoxins

In another aspect, the present invention features a human anti-HER2/neu monoclonal antibody, or a fragment thereof, conjugated to a therapeutic moiety, such as a cytotoxin, a drug or a radioisotope. When conjugated to a cytotoxin, these antibody conjugates are referred to as "immunotoxins." A cytotoxin or cytotoxic agent includes any agent that is detrimental to (e.g., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, cannustinc (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). An antibody of the present invention can be conjugated to a radioisotope, e.g., radioactive iodine, to generate cytotoxic radiopharmaceuticals for treating a HER2/neu-related disorder, such as a cancer.

The antibody conjugates of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-γ; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

### V. Pharmaceutical Compositions

In another aspect, the present invention provides a composition, e.g., a pharmaceutical composition, containing one or a combination of human monoclonal antibodies, or antigen-binding portion(s) thereof, of the present invention, formulated together with a pharmaceutically acceptable carrier. In a preferred embodiment, the compositions include a combination of multiple (e.g., two or more) isolated human antibodies or antigen-binding portions thereof of the invention. Preferably, each of the antibodies or antigen-binding portions thereof of the composition binds to a distinct, preselected epitope of HER2/neu.

In one embodiment, human anti-HER2/neu monoclonal antibodies having complementary activities are used in combination, e.g., as a pharmaceutial composition, comprising two or more human anti-HER2/neu monoclonal antibodies. For example, a human monoclonal antibody that mediates highly effective killing of target cells in the presence of effector cells can be combined with another human monoclonal antibody that inhibits the growth of cells expressing HER2/neu.

In another embodiment, the composition comprises one or a combination of bispecific or multispecific molecules of the invention (e.g., which contains at least one binding specificity for an Fc receptor and at least one binding specificity for HER2/neu).

Pharmaceutical compositions of the invention also can be administered in combination therapy, i.e., combined with other agents. For example, the combination therapy can include a composition of the present invention with at least one anti-tumor agent or other conventional therapy.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, i.e., antibody, bispecific and multispecific molecule, may be coated in a material to protect the compound from the action of acids and other natural conditions that may inactivate the compound.

A "pharmaceutically acceptable salt" refers to a salt that retains the desired biological activity of the parent compound and does not impart any undesired toxicological effects (see e.g., Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A composition of the present invention can be administered by a variety of methods known in the art. As will be appreciated by the skilled artisan, the route and/or mode of administration will vary depending upon the desired results. The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J.R. Robinson, ed., Marcel Dekker, Inc., New York, 1978.

To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al. (1984) J. Neuroimmunol. 7:27).

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic compositions, formulations of the present invention include those suitable for oral, nasal, topical (including buccal and sublingual), rectal, vaginal and/or parenteral administration. The formulations may conveniently be presented in unit dosage form and may be prepared by any methods known in the art of pharmacy. The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.01 per cent to about ninety-nine percent of active ingredient, preferably from about 0.1 per cent to about 70 per cent, most preferably from about 1 per cent to about 30 per cent.

Formulations of the present invention which are suitable for vaginal administration also include pessaries, tampons, creams, gels, pastes, foams or spray formulations containing such carriers as are known in the art to be appropriate. Dosage forms for the topical or transdermal administration of compositions of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

When the compounds of the present invention are administered as pharmaceuticals, to humans and animals, they can be given alone or as a pharmaceutical composition containing, for example, 0.01 to 99.5% (more preferably, 0.1 to 90%) of active ingredient in combination with a pharmaceutically acceptable carrier.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. In general, a suitable daily dose of a compositions of the invention will be that amount of the compound which is the lowest dose effective to produce a therapeutic effect. Such an effective dose will generally depend upon the factors described above. It is preferred that administration be intravenous, intramuscular, intraperitoneal, or subcutaneous, preferably administered proximal to the site of the target. If desired, the effective daily dose of a therapeutic compositions may be administered as two, three, four, five, six or more sub-doses administered separately at appropriate intervals throughout the day, optionally, in unit dosage forms. While it is possible for a compound of the present invention to be administered alone, it is preferable to administer the compound as a pharmaceutical formulation (composition).

Therapeutic compositions can be administered with medical devices known in the art. For example, in a preferred embodiment, a therapeutic composition of the invention can be administered with a needleless hypodermic injection device, such as the devices disclosed in U.S. Patent Nos. 5,399,163, 5,383,851, 5,312,335, 5,064,413, 4,941,880, 4,790,824, or 4,596,556. Examples of well-known implants and modules useful in the present invention include: U.S. Patent No. 4,487,603, which discloses an implantable micro-infusion pump for dispensing medication at a controlled rate; U.S. Patent No. 4.,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system.

Many other such implants, delivery systems, and modules are known to those skilled in the art.

In certain embodiments, the human monoclonal antibodies of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, e.g., U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, e.g., U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J Physiol. 1233:134), different species of which may comprise the formulations of the inventions, as well as components of the invented molecules; p120 (Schreier et al. (1994) J Biol. Chem. 269:9090); see also K. Keinanen; M.L. Laukkanen (1994) FEBS Lett. 346:123; J.J. Killion; I.J. Fidler (1994) Immunomethods 4:273. In one embodiment of the invention, the therapeutic compounds of the invention are formulated in liposomes; in a more preferred embodiment, the liposomes include a targeting moiety. In a most preferred embodiment, the therapeutic compounds in the liposomes are delivered by bolus injection to a site proximal to the tumor or infection. The composition must be fluid to the extent that easy syringability exists. It must be stable under the conditions of manufacture and storage and must be preserved against the contaminating action of microorganisms such as bacteria and fungi.

A "therapeutically effective dosage" preferably inhibits tumor growth by at least about 20%, more preferably by at least about 40%, even more preferably by at least about 60%, and still more preferably by at least about 80% relative to untreated subjects. The ability of a compound to inhibit cancer can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

The composition must be sterile and fluid to the extent that the composition is deliverable by syringe. In addition to water, the carrier can be an isotonic buffered saline solution, ethanol, polyol (for example, glycerol, propylene glycol, and liquid polyetheylene glycol, and the like), and suitable mixtures thereof. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as manitol or sorbitol, and sodium chloride in the composition. Long-term absorption of the injectable compositions can be brought about by including in the composition an agent which delays absorption, for example, aluminum monostearate or gelatin.

When the active compound is suitably protected, as described above, the compound may be orally administered, for example, with an inert diluent or an assimilable edible carrier.

### VI. Uses of the Invention

The compositions (e.g., human monoclonal antibodies to HER2/neu and derivatives/conjugates thereof) of the present invention have *in vitro* and *in vivo* diagnostic and therapeutic utilities. For example, these molecules can be administered to cells in culture, e.g. *in vitro* or *ex vivo,* or in a subject, e.g., *in vivo,* to treat, prevent or diagnose a variety of disorders. As used herein, the term "subject" is intended to include human and non-human animals. Preferred human animals include a human patient having disorder characterized by expression, typically aberrant expression (e.g., overexprcssion) of HER2/neu. For example, the compositions of the present invention can be used to treat a subject with a tumorigenic disorder, e.g., a disorder characterized by the presence of tumor cells expressing HER2/neu, including, for example, cells of an adenocarcinoma, e.g., salivary gland, stomach and kidney, mammary gland carcinoma, lung carcinoma, squamous cell carcinoma, and ovarian cancer cells. The term "non-human animals" of the invention includes all vertebrates, e.g., mammals and non-mammals, such as non-human primates, sheep, dog, cow, chickens, amphibians, reptiles, etc.

The compositions (e.g., human antibodies, multispecific and bispecific molecules) of the invention can be initially tested for binding activity associated with therapeutic or diagnostic use *in vitro.* For example, compositions of the invention can be tested using the ELISA and flow cytometric assays described in the Examples below. Moreover, the activity of these molecules in triggering at least one effector-mediated effector cell activity, including cytolysis of cells expressing HER2/neu can be assayed. Protocols for assaying for effector cell-mediated cytolysis are described in the Examples below.

The compositions (e.g., human antibodies, multispecific and bispecific molecules) of the invention have additional utility in therapy and diagnosis of HER2/neu-related diseases. For example, the human monoclonal antibodies, the multispecific or bispecific molecules can be used, for example, to elicit *in vivo* or *in vitro* one or more of the following biological activities: to opsonize a cell expressing HER2/neu; to mediate phagocytosis or cytolysis of a cell expressing HER2/neu in the presence of human effector cells; or to inhibit the growth of a cell expressing HER2/neu.

In a particular embodiment, the human antibodies and derivatives thereof are used *in vivo* to treat, prevent or diagnose a variety of HER2/neu-related diseases. Examples of HER2/neu-related diseases include a variety of cancers, such as adenocarcinoma, e.g. salivary gland, stomach and kidney, mammary gland carcinoma, lung carcinoma, squamous cell carcinoma, and ovarian cancer.

Methods of administering the compositions (e.g., human antibodies, multispecific and bispecific molecules) of the invention are known in the art. Suitable dosages of the molecules used will depend on the age and weight of the subject and the particular drug used. The molecules can be coupled to radionuclides, such as 131I, 90Y, 105Rh, etc., as described in Goldenberg, D.M. et al. (1981) Cancer Res. 41: 4354-4360, and in EP 0365 997. The compositions (e.g., human antibodies, multispecific and bispecific molecules) of the invention can also be coupled to anti-infectious agents.

Target-specific effector cells, e.g., effector cells linked to compositions (e.g., human antibodies, multispecific and bispecific molecules) of the invention can also be used as therapeutic agents. Effector cells for targeting can be human leukocytes such as macrophages, neutrophils or monocytes. Other cells include and other IgG- or IgA-receptor bearing cells. If desired, effector cells can be obtained from the subject to be treated. The target-specific effector cells, can be administered as a suspension of cells in a physiologically acceptable solution. The number of cells administered can be in the order of 10⁸-10⁹ but will vary depending on the therapeutic purpose. In general, the amount will be sufficient to obtain localization at the target cell, e.g., a tumor cell expressing HER2/neu, and to effect cell killing by, e.g., phagocytosis. Routes of administration can also vary.

Therapy with target-specific effector cells can be performed in conjunction with other techniques for removal of targeted cells. For example, anti-tumor therapy using the compositions (e.g., human antibodies, multispecific and bispecific molecules) of the invention and/or effector cells armed with these compositions can be used in conjunction with chemotherapy. Additionally, combination immunotherapy may be used to direct two distinct cytotoxic effector populations toward tumor cell rejection. For example, anti-HER2/neu antibodies linked to anti-Fc-gammaRI or anti-T3 may be used in conjunction with IgG- or IgA-receptor specific binding agents.

Bispecific and multispecific molecules of the invention can also be used to modulate FcγR or FcαR levels on effector cells, such as by capping and elimination of receptors on the cell surface. Mixtures of anti-Fc receptors can also be used for this purpose.

The compositions (e.g., human antibodies, multispecific and bispecific molecules) of the invention which have complement binding sites, such as portions from IgG1, -2, or -3 or IgM which bind complement, can also be used in the presence of complement. In one embodiment, *ex vivo* treatment of a population of cells comprising target cells with a binding agent of the invention and appropriate effector cells can be supplemented by the addition of complement or serum containing complement. Phagocytosis of target cells coated with a binding agent of the invention can be improved by binding of complement proteins. In another embodiment target cells coated with the compositions (e.g., human antibodies, multispecific and bispecific molecules) of the invention can also be lysed by complement.

The compositions (e.g., human antibodies, multispecific and bispecific molecules) of the invention can also be administered together with complement.

These compositions are advantageous in that the complement is located in close proximity to the human antibodies, multispecific or bispecific molecules. Alternatively, the human antibodies, multispecific or bispecific molecules of the invention and the complement or serum can be administered separately.

Kits comprising the compositions can be provided (e.g., human antibodies, multispecific and bispecific molecules) of the invention and instructions for use. The kit can further contain a least one additional reagent, such as complement, or one or more additional human antibodies of the invention (e.g., a human antibody having a complementary activity which binds to an epitope in the HER2/neu antigen distinct from the first human antibody).

In other embodiments, the subject can be additionally treated with an agent that modulates, e.g., enhances or inhibits, the expression or activity of Fcγ or Fcα receptors, by for example, treating the subject with a cytokine. Preferred cytokines for administration during treatment with the multispecific molecule include of granulocyte colony-stimulating factor (G-CSF), granulocyte- macrophage colony-stimulating factor (GM-CSF), interferon-γ (IFN-γ), and tumor necrosis factor (TNF).

The compositions (e.g., human antibodies, multispecific and bispecific molecules) of the invention can also be used to target cells expressing FcγR or HER2/neu, for example for labeling such cells. For such use, the binding agent can be linked to a molecule that can be detected. Thus, methods can be provided for localizing *ex vivo* or *in vitro* cells expressing Fc receptors, such as FcγR, or HER2/neu. The detectable label can be, e.g., a radioisotope, a fluorescent compound, an enzyme, or an enzyme co-factor.

Methods can be provided for detecting the presence of HER2/neu antigen in a sample, or measuring the amount of HER2/neu antigen, comprising contacting the sample, and a control sample, with a human monoclonal antibody, or an antigen binding portion thereof, which specifically binds to HER2/neu, under conditions that allow for formation of a complex between the antibody or portion thereof and HER2/neu. The formation of a complex is then detected, wherein a difference complex formation between the sample compared to the control sample is indicative the presence of HER2/neu antigen in the sample.

A method can be provided for detecting the presence or quantifying the amount of Fc-expressing cells *in vivo* or *in vitro.* The method comprises (i) administering to a subject a composition (e.g., a multi- or bispecific molecule) of the invention or a fragment thereof, conjugated to a detectable marker; (ii) exposing the subject to a means for detecting said detectable marker to identify areas containing Fc-expressing cells.

Other embodiments of the present invention are described in the following Examples.

The present invention is further illustrated by the following examples which should not be construed as further limiting.

### EXAMPLES

### Example 1 Generation of Cmu targeted mice

### Construction of a CMD targeting vector

The plasmid pICEmu contains an EcoRI/XhoI fragment of the murine Ig heavy chain locus, spanning the mu gene, that was obtained from a Balb/C genomic lambda phage library (Marcu et al. Cell 22: 187, 1980). This genomic fragment was subcloned into the XhoI/EcoRI sites of the plasmid pICEM19H (Marsh et al; Gene 32, 481-485, 1984). The heavy chain sequences included in pICEmu extend downstream of the EcoRI site located just 3' of the mu intronic enhancer, to the XhoI site located approximately 1 kb downstream of the last transmembrane exon of the mu gene; however, much of the mu switch repeat region has been deleted by passage in *E. coli.*

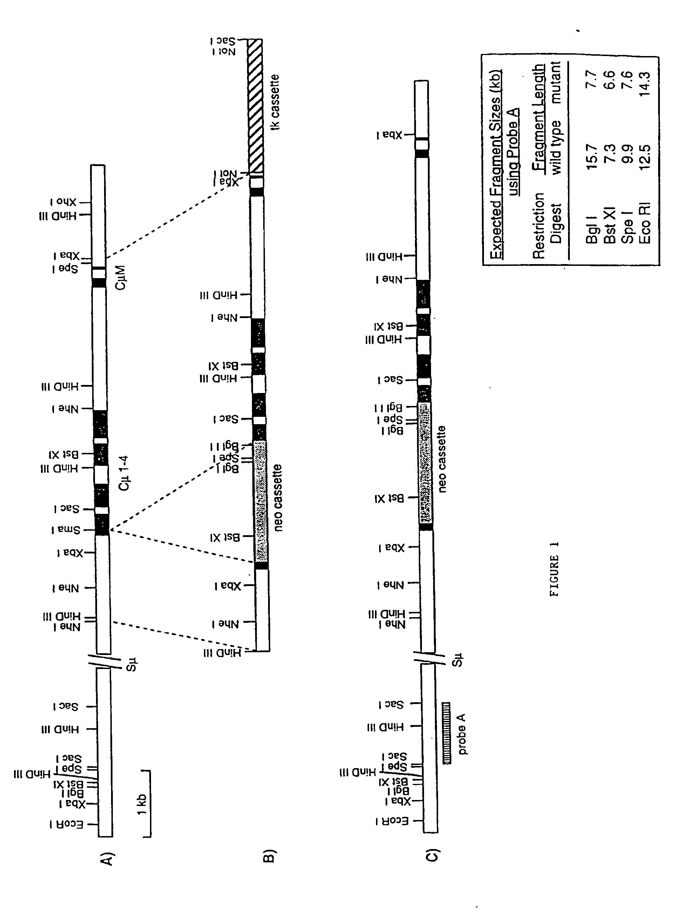

The targeting vector was constructed as follows (see Figure 1). A 1.3 kb HindIII/SmaI fragment was excised from pICEmu and subcloned into HindIII/Smal digested pBluescript (Stratagene, La Jolla, CA). This pICEmu fragment extends from the HindIII site located approximately 1 kb 5' of Cmul to the SmaI site located within Cmul. The resulting plasmid was digested with SmaI/SpeI and the approximately 4 kb SmaI/XbaI fragment from pICEmu, extending from the Sma I site in Cmu1 3' to the XbaI site located just downstream of the last Cmu exon, was inserted. The resulting plasmid, pTAR1, was linearized at the SmaI site, and a neo expression cassette inserted. This cassette consists of the neo gene under the transcriptional control of the mouse phosphoglycerate kinase (pgk) promoter (XbaI/TaqI fragment; Adra et al. (1987) Gene 60: 65-74) and containing the pgk polyadenylation site (PvuII/HindIII fragment; Boer et al. (1990) Biochemical Genetics 28: 299-308). This cassette was obtained from the plasmid pKJ 1 (described by Tybulewicz et al. (1991) Cell 65: 1153-1163) from which the neo cassette was excised as an EcoRI/HindIII fragment and subcloned into EcoRI/HindIII digested pGEM-7Zf (+) to generate pGEM-7 (KJ1). The neo cassette was excised from pGEM-7 (KJ1) by EcoRI/SalI digestion, blunt ended and subcloned into the SmaI site of the plasmid pTAR1, in the opposite orientation of the genomic Cmu sequences. The resulting plasmid was linearized with Not I, and a herpes simplex virus thymidine kinase (tk) cassette was inserted to allow for enrichment of ES clones bearing homologous recombinants, as described by Mansour et al. (1988) Nature 336: 348-352. This cassette consists of the coding sequences of the tk gene bracketed by the mouse pgk promoter and polyadenylation site, as described by Tybulewicz et al. (1991) Cell 65: 1153-1163. The resulting CMD targeting vector contains a total of approximately 5.3 kb of homology to the heavy chain locus and is designed to generate a mutant mu gene into which has been inserted a neo expression cassette in the unique SmaI site of the first Cmu exon. The targeting vector was linearized with PvuI, which cuts within plasmid sequences, prior to electroporation into ES cells.

### Generation and analysis of targeted ES cells

AB-1 ES cells (McMahon, A. P. and Bradley, A., (1990) Cell 62: 1073-1085) were grown on mitotically inactive SNL76/7 cell feeder layers (ibid.) essentially as described (Robertson, E. J. (1987) in Teratocarcinomas and Embryonic Stem Cells: a Practical Approach (E. J. Robertson, ed.) Oxford: IRL Press, p. 71-112). The linearized CMD targeting vector was electroporated into AB-1 cells by the methods described Hasty *et al.* (Hasty, P. R. et al. (1991) Nature 350: 243-246). Electroporated cells were plated into 100 mm dishes at a density of 1-2 x 10⁶ cells/dish. After 24 hours, G418 (200 micrograms/ml of active component) and FIAU (5 x 10⁻⁷ M) were added to the medium, and drug-resistant clones were allowed to develop over 8-9 days. Clones were picked, trypsinized, divided into two portions, and further expanded. Half of the cells derived from each clone were then frozen and the other half analyzed for homologous recombination between vector and target sequences.

DNA analysis was carried out by Southern blot hybridization. DNA was isolated from the clones as described Laird *et al.* (Laird, P. W. et al., (1991) Nucleic Acids Res. 19 : 4293). Isolated genomic DNA was digested with SpeI and probed with a 915 bp SacI fragment, probe A (see Figure 1), which hybridizes to a sequence between the mu intronic enhancer and the mu switch region. Probe A detects a 9.9 kb SpeI fragment from the wild type locus, and a diagnostic 7.6 kb band from a mu locus which has homologously recombined with the CMD targeting vector (the neo expression cassette contains a SpeI site). Of 1132 G418 and FIAU resistant clones screened by Southern blot analysis, 3 displayed the 7.6 kb Spe I band indicative of homologous recombination at the mu locus. These 3 clones were further digested with the enzymes BglI, BstXI, and EcoRI to verify that the vector integrated homologously into the mu gene. When hybridized with probe A, Southern blots of wild type DNA digested with BglI, BstXI, or EcoRI produce fragments of 15.7, 7.3, and 12.5 kb, respectively, whereas the presence of a targeted mu allele is indicated by fragments of 7.7, 6.6, and 14.3 kb, respectively. All 3 positive clones detected by the SpeI digest showed the expected BglI, BstXI, and EcoRI restriction fragments diagnostic of insertion of the neo cassette into the Cmul exon.

### Generation of mice bearing the mutated mu gene

The three targeted ES clones, designated number 264, 272, and 408, were thawed and injected into C57BL/6J blastocysts as described by Bradley (Bradley, A. (1987) in Teratocarcinomas and Embryonic Stem Cells: a Practical Approach. (E. J. Robertson, ed.) Oxford: IRL Press, p. 113-151). Injected blastocysts were transferred into the uteri of pseudopregnant females to generate chimeric mice representing a mixture of cells derived from the input ES cells and the host blastocyst. The extent of ES cell contribution to the chimera can be visually estimated by the amount of agouti coat coloration, derived from the ES cell line, on the black C57BL/6J background. Clones 272 and 408 produced only low percentage chimeras (i.e. low percentage of agouti pigmentation) but clone 264 produced high percentage male chimeras. These chimeras were bred with C57BL/6J females and agouti offspring were generated, indicative of germline transmission of the ES cell genome. Screening for the targeted mu gene was carried out by Southern blot analysis of BglI digested DNA from tail biopsies (as described above for analysis of ES cell DNA). Approximately 50% of the agouti offspring showed a hybridizing BglI band of 7.7 kb in addition to the wild type band of 15.7 kb, demonstrating a germline transmission of the targeted mu gene.

### Analysis of transgenic mice for functional inactivation of mu gene

To determine whether the insertion of the neo cassette into Cmul has inactivated the Ig heavy chain gene, a clone 264 chimera was bred with a mouse homozygous for the JHD mutation, which inactivates heavy chain expression as a result of deletion of the JH gene segments (Chen et al, (1993) Immunol. 5: 647-656). Four agouti offspring were generated. Serum was obtained from these animals at the age of 1 month and assayed by ELISA for the presence of murine IgM. Two of the four offspring were completely lacking IgM (see Table 1). Genotyping of the four animals by Southern blot analysis of DNA from tail biopsies by BglI digestion and hybridization with probe A (see Figure 1), and by StuI digestion and hybridization with a 475 bp EcoRI/StuI fragment (ibid.) demonstrated that the animals which fail to express serum IgM are those in which one allele of the heavy chain locus carries the JHD mutation, the other allele the Cmu1 mutation. Mice heterozygous for the JHD mutation display wild type levels of serum Ig. These data demonstrate that the Cmu1 mutation inactivates expression of the mu gene.

**TABLE 1**

| **Mouse** | **Serum IgM** **(micrograms/ml)** | **Ig H chain genotype** |
|---|---|---|
| 42 | <0.002 | CMD/JHD |
| 43 | 196 | +/JHD |
| 44 | <0.002 | CMD/JHD |
| 45 | 174 | +/JHD |
| 129 x BL6 F1 | 153 | +/+ |
| JHD | <0.002 | JHD/JHD |

Table 1 shows the levels of serum IgM, detected by ELISA, for mice carrying both the CMD and JHD mutations (CMD/JHD), for mice heterozygous for the JHD mutation (+/JHD), for wild type (129Sv x C57BL/6J)F1 mice (+/+), and for B cell deficient mice homozygous for the JHD mutation (JHD/JHD).

### Example 2 Generation of HCO12 transgenic mice

### The HCO12 human heavy chain transgene

The HCO12 transgene was generated by coinjection of the 80 kb insert of pHC2 (Taylor et al., 1994, Int. Immunol., 6: 579-591) and the 25 kb insert of pVx6. The plasmid pVx6 was constructed as described below.

An 8.5 kb HindIII/SalI DNA fragment, comprising the germline human VH1-18 (DP-14) gene together with approximately 2.5 kb of 5' flanking, and 5 kb of 3' flanking genomic sequence was subcloned into the plasmid vector pSP72 (Promega, Madison, WI) to generate the plasmid p343.7.16. A 7 kb BamHI/HindIII DNA fragment, comprising the germline human VH5-51 (DP-73) gene together with approximately 5 kb of 5' flanking and 1 kb of 3' flanking genomic sequence, was cloned into the pBR322 based plasmid cloning vector pGP1f (Taylor et al. 1992, Nucleic Acids Res. 20: 6287-6295), to generate the plasmid p251f. A new cloning vector derived from pGP1f, pGP1k (SEQ ID NO: 13), was digested with EcoRV/BamHI, and ligated to a 10 kb EcoRV/BamHI DNA fragment, comprising the germline human VH3-23 (DP47) gene together with approximately 4 kb of 5' flanking and 5 kb of 3' flanking genomic sequence. The resulting plasmid, p112.2RR.7, was digested with BamHI/SalI and ligated with the 7 kb purified BamHI/SalI insert of p251f. The resulting plasmid, pVx4, was digested with XhoI and ligated with the 8.5 kb XhoI/SalI insert of p343.7.16.

A clone was obtained with the VH1-18 gene in the same orientation as the other two V genes. This clone, designated pVx6, was then digested with NotI and the purified 26 kb insert coinjected--together with the purified 80 kb NotI insert of pHC2 at a 1:1 molar ratio--into the pronuclei of one-half day (C57BL/6J x DBA/2J)F2 embryos as described by Hogan *et al.* (B. Hogan et al., Manipulating the Mouse Embryo, A Laboratory Manual, 2nd edition, 1994, Cold Spring Harbor Laboratory Press, Plainview NY). Three independent lines of transgenic mice comprising sequences from both Vx6 and HC2 were established from mice that developed from the injected embryos. These lines are designated (HCO12)14881, (HCO12)15083, and (HCO12)15087. Each of the three lines were then bred with mice comprising the CMD mutation described in Example 1, the JKD mutation (Chen et al. 1993, EMBO J. 12: 811-820), and the (KCo5)9272 transgene (Fishwild et al. 1996, Nature Biotechnology 14: 845-851). The resulting mice express human heavy and kappa light chain transgenes in a background homozygous for disruption of the endogenous mouse heavy and kappa light chain loci.

### Example 3 Production of Human Monoclonal Antibodies and Bispecifics Against HER2/neu

Human anti-HER2/neu monoclonal antibodies were generated by immunizing the HCO7 and HCO12 strains of HuMAb mice with HER2/neu antigen derived from three sources: 1) human breast carcinoma cells which express high levels of HER2/neu, SKBR-3 and BT-474, grown under standard conditions, harvested and washed with PBS; 2) Lysates from SKBR-3 cells immunoprecipitated with a murine anti-HER2/neu monoclonal antibody and anti-murine IgG sepharose; and 3) HER2/neu shed from SKBR-3 cells and purified by affinity chromatography using a murine anti-HER2/neu monoclonal antibody. HCO7 HuMAb mice were generated as described in U.S. Patent Nos. 5,545,806, 5,625,825, and 5,545,807, the entire disclosures of which are hereby incorporated by reference.

In particular, HCO7 and HCO12 mice were initially immunized with antigens emulsified in Complete Freund's Adjuvant. In subsequent immunizations, antigens were mixed with Incomplete Freund's Adjuvant. Finally, intravenous administration of antigen prior to splenectomy was performed with purified antigen, and no adjuvant. Mice were immunized every 2-3 weeks. After the third and following immunizations, the human IgG anti-HER2/neu titer was determined by ELISA (as described below). Mice that developed an anti-HER2/neu IgG response were administered purified antigen i.v. for three days or both three and four days prior to splenectomy. The spleens from responding mice were harvested and dispersed into single cells.

To generate hybridomas producing anti-HER2/neu antibodies, splenocytes from mice with plasma containing anti-HER2/neu antibodies were fused with P3X63-Ag8.653 cells (deposited with the ATCC under designation ATCC CRL 1580 nonsecreting mouse myeloma cells) and PEG. After hybridomas grew out (about 10-14 days) each well containing hybridomas was screened for the production of human IgG using an anti-human IgG ELISA. Positive hybridomas were further tested for antigen specificity using a HER2/neu ELISA.

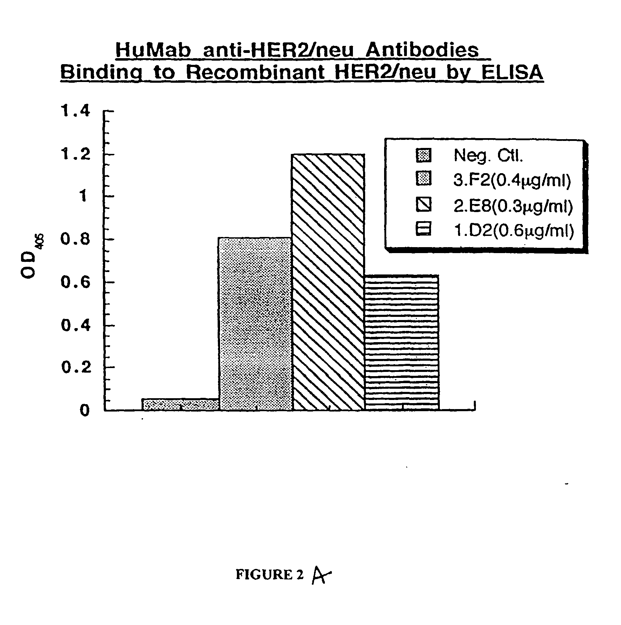

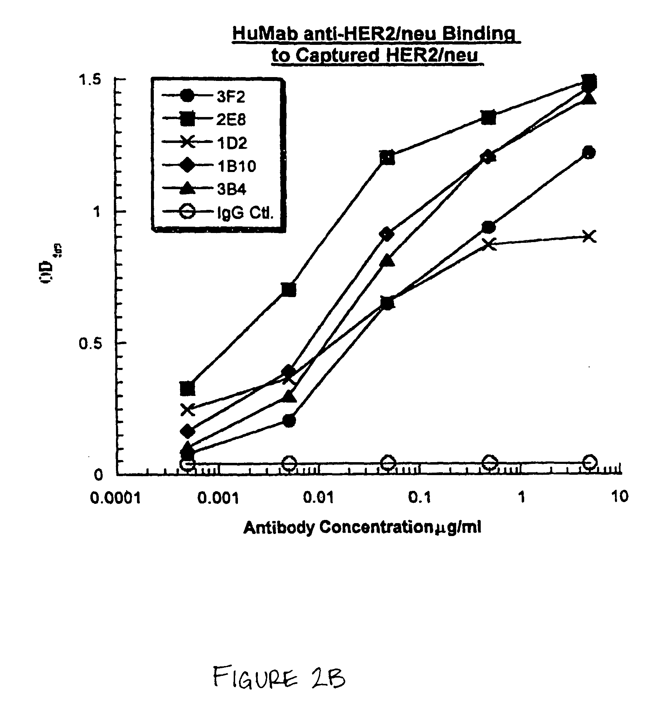

Briefly, microtiter plates were coated with purified HER2/neu at 0.25 µg/ml in PBS, and then blocked with 5% bovine serum albumin in PBS. Supernatants from the anti-HER2/neu producing hybridomas were added to each well and incubated for 1-2 hours at 37 °C. The plates were washed with PBS/Tween and then incubated with a goat-anti-human IgG Fc-specific polyclonal reagent conjugated to alkaline phosphatase for 1 hour at 37 °C. After washing, the plates were exposed to pNPP and the samples read with a microplate reader at a wavelength of 405nm. Figure 2A depicts the binding of three supernatants from the hybridoma cultures termed 3.F2, 2.E8 and 1.D2 to recombinant HER2/neu as measured by ELISA. Significant binding was detected with the three anti-HER2/neu hybridoma supernatants shown as compared to a media control.

Several hybridomas that secreted human IgG antibodies with specificity for HER2/neu were subcloned and expanded for purification of the human monoclonal antibodies. Monoclonal antibodies were isolated from supernatants of hybridoma cultures grown in spinner flasks in a humidified incubator containing 5% CO2. Antibodies were purified by chromatography on a Protein A-agarose column according to the manufacturer's specifications (Pierce, Rockford IL).

Microtiter plates were coated with mouse anti-HER2/neu antibody, blocked with 5% BSA, and then incubated with the supernatant from overgrown SKBR-3 cells, thus capturing the HER2/neu shed by the cells. The plates were then incubated with varying concentrations of human anti-HER2/neumonoclonal antibodies or an isotype control (human IgG1). The human anti-HER2/neu antibodies that bound to immobolized HER2/neu were detected as described above. In particular, Figure 2B shows binding of antibodies 3.F2, 2.E8, 1.D2, 1.B10, 3.B4 to recombinant HER2/neu as measured by ELISA.

Hybridomas which produced human IgG that bound HER2/neu by ELISA were further characterized by ELISA and flow cytometry for isotype, binding to tumor cells expressing HER2/neu, and lack of binding to HER2/neu-negative cells, as described below. Hybridomas with supernatants exhibiting these properties were subcloned, and their antibodies further characterized after purification by protein A affinity chromatography.

Twelve hybridomas that were screened produce human IgG1κ antibodies that specifically bind to HER2/neu as assessed by ELISA and flow cytometry (3.F2, 2.E8, 1.D2, 1.B10, 3.B4, 1.F11, 3.D11, 3.D6, 1.H9, 2.G7, 3.E8 and 2.E11). Five of these monoclonal antibodies (3.F2, 2.E8, 1.D2, 1.B10 and 3.B4) were purified and found to bind purified HER2/neu and human tumor cells expressing HER2/neu. These five antibodies were shown to mediate cytolytic killing of human tumor cells expressing HER2/neu and inhibit human tumor cell growth *in vitro.*

In addition, a bispecific molecule, referred to as 14.1 x 3.F2, was made by chemical conjugation of the Fab'₂ fragments from the human anti-CD89 antibody 14.1 and the human anti-HER2/neu antibody 3.F2 via disulfide bonds using standard cross-linking procedures (Figure 13).

### Example 4 Characterization of Human Monoclonal Antibodies and Bispecifics Against HER2/neu

### Binding of human anti-HER2/neu antibodies and Bispecifics to tumor cells

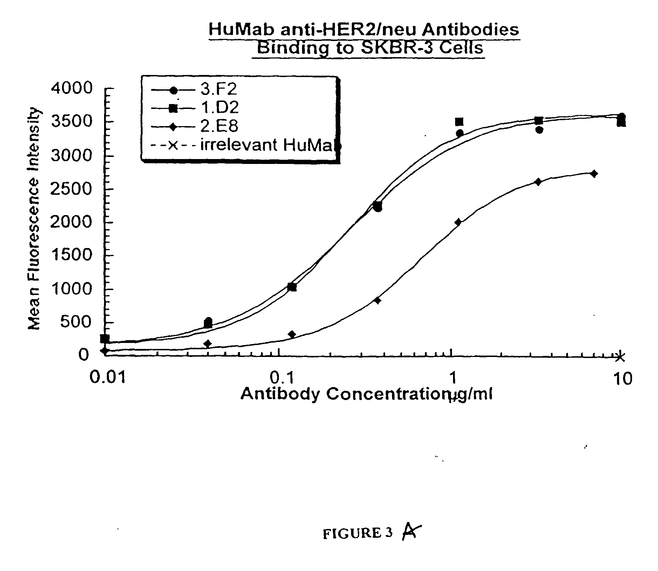

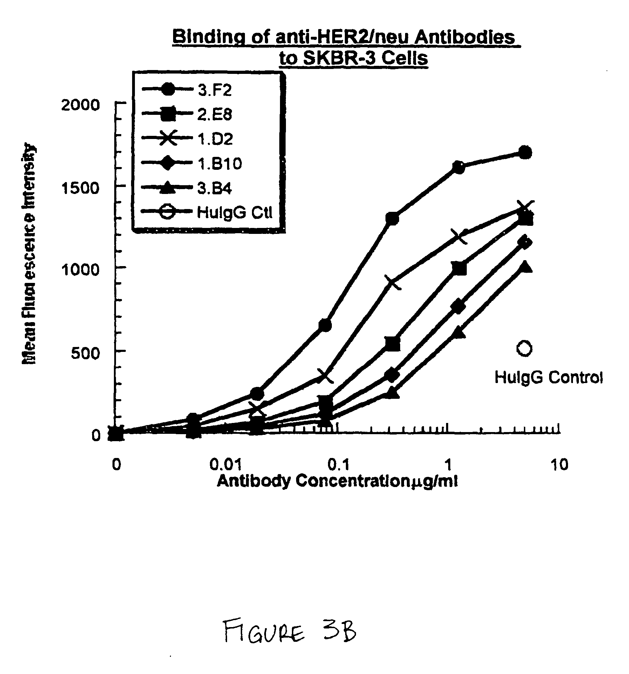

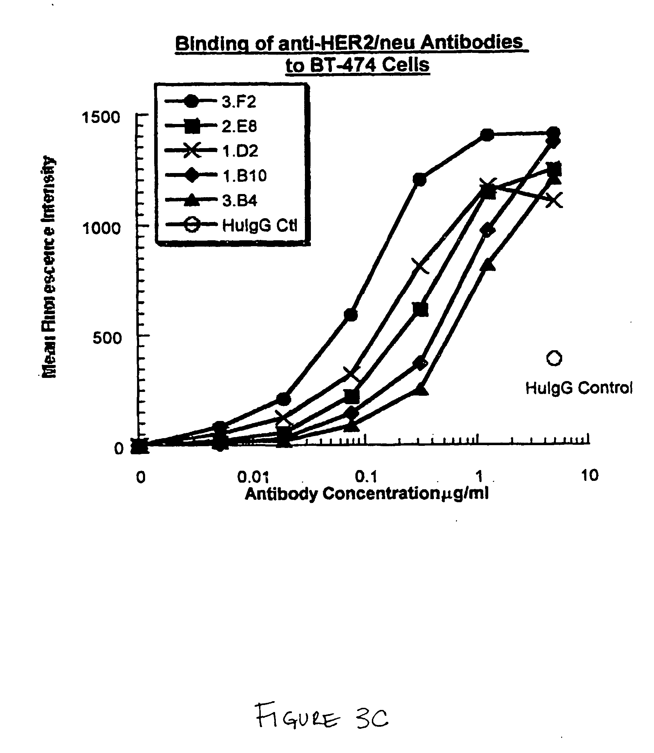

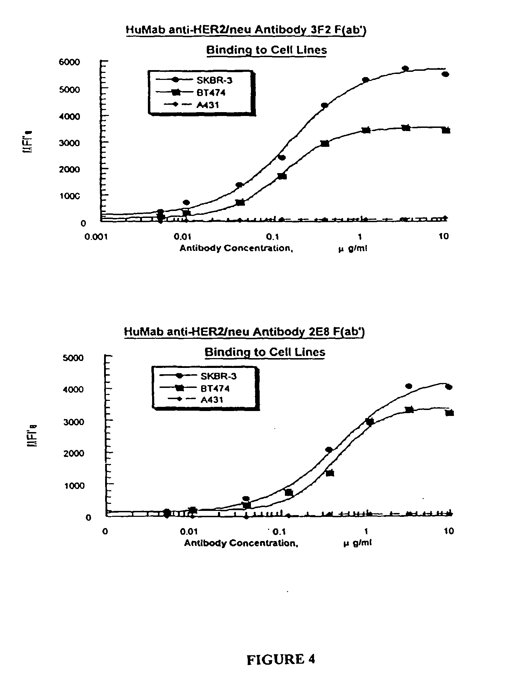

Monoclonal antibodies purified from five hybridoma supernatants that showed significant binding to HER2/neu as detected by ELISA (3.F2, 2.E8, 1.D2, 1.B10 and 3.B4) were further tested for the ability to bind to tumor cells that express high levels of HER2/neu. Briefly, tumor cells (e.g., SKBR-3 or BT-474) were incubated with either antibodies or antibody F(ab')₂ fragments and washed to remove unbound antibody. Human anti-HER2/neu antibody bound to the cells was detected using a fluorochrome labeled goat-anti human F(ab')₂ and the mean fluorescence intensity of the samples determined using flow cytometry. As shown in Figures 3A and 3B, the anti-HER2/neu antibodies 3.F2, 2.E8, 1.D2, 1.B10 and 3.B4 exhibit significant binding to SKBR-3 cells expressing HER2/neu, as compared to a control human antibody. Similarly, Figure 3C shows binding of antibodies 3.F2, 2.E8, 1.D2, 1.B10 and 3.B4 to BT-474 tumor cells which express HER2/neu on their surface. Figure 4 demonstrates the specificity of the 3.F2 and 2.E8 antibodies for HER2/neu. The anti-HER2/neu antibodies 3.F2 and 2.E8 exhibit significant, specific binding to the HER2/neu expressing tumor cell lines SKBR-3 and BT-474, whereas they do not bind significantly to the A431 tumor cell line expressing high levels of the EGF receptor (c-erbB1) which is in the same family as the HER2/neu receptor (c-erbB2). Bispecific molecules including the human anti-HER2/neu antibodies or antibody fragments can be tested for binding in this same manner.

### Inhibition of human tumor cell growth using human anti-HER2/neu antibodies and Bispecifics

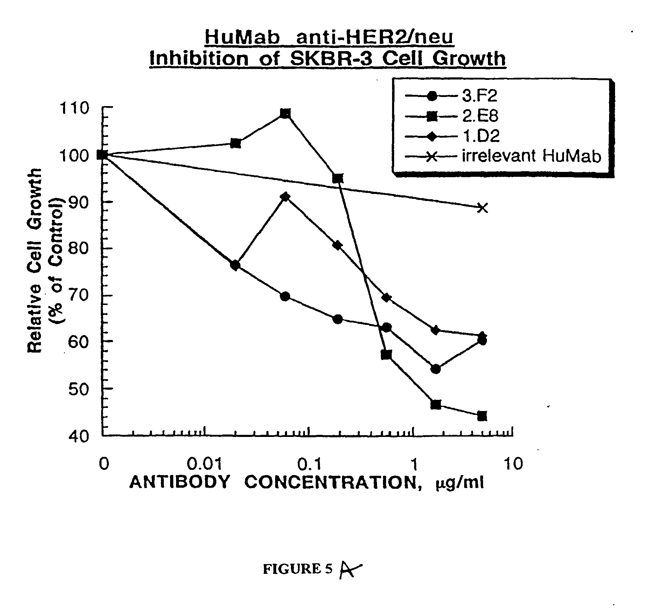

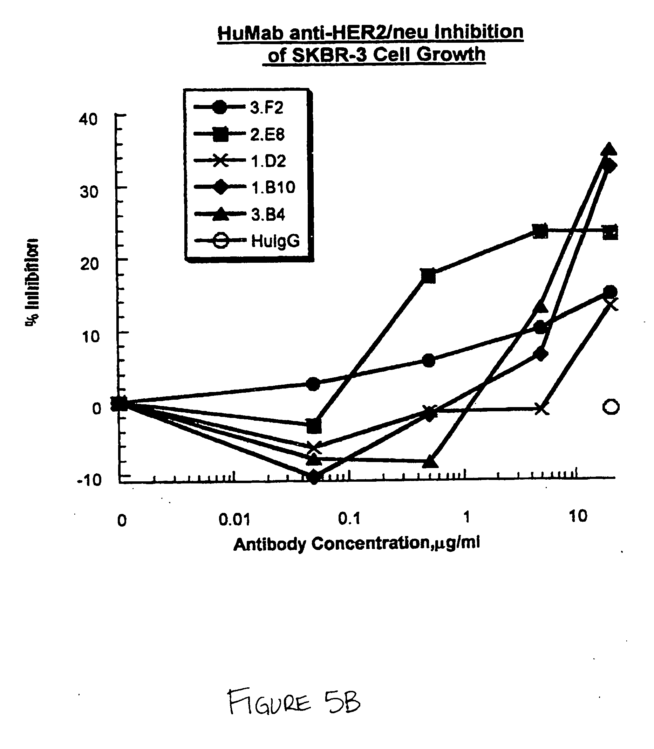

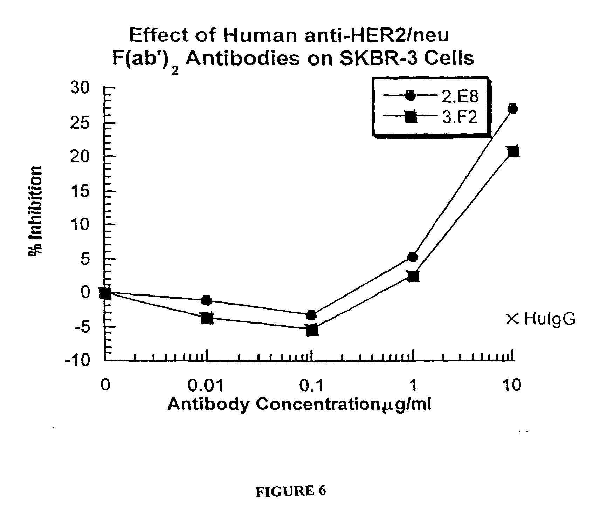

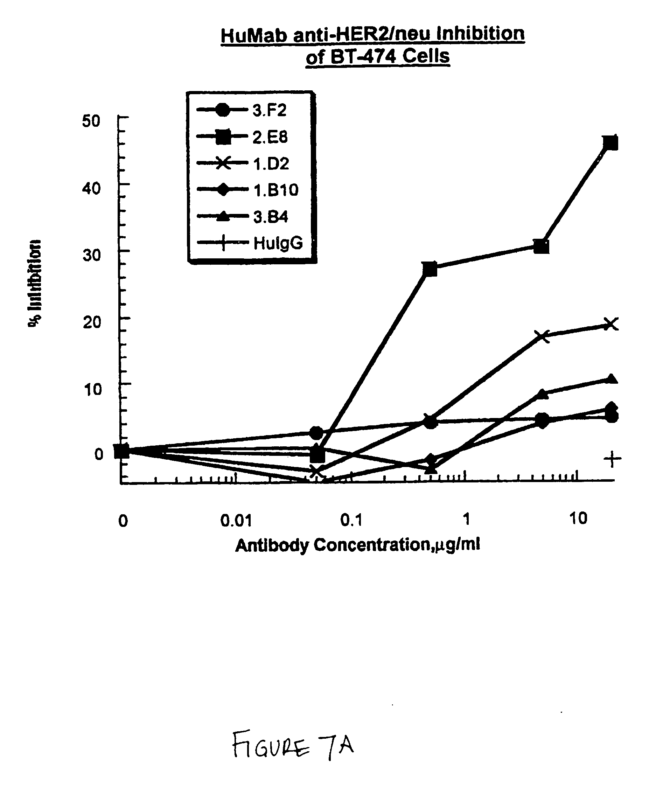

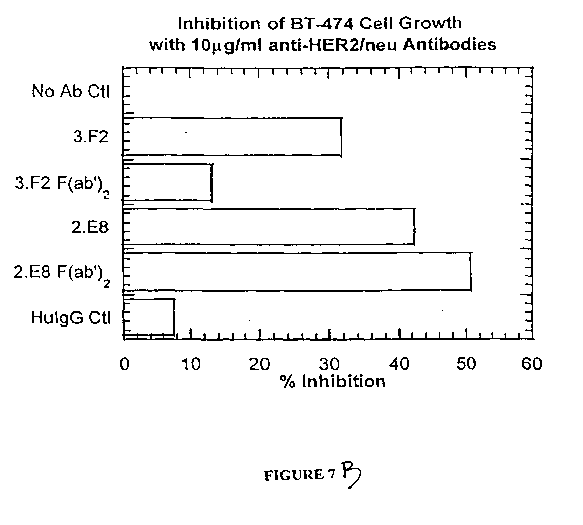

The antibodies that showed specific binding to HER2/neu, as demonstrated above, were then tested for the ability to inhibit the growth of human tumor cells expressing cell surface HER2/neu. Briefly, purified antibodies were incubated with the tumor cells for 6 days under normal growth conditions, and cell density was measured by crystal violet staining. As demonstrated in Figures 5A amd 5B, treatment with purified anti-HER2/neu antibodies 3.F2, 2.E8, 1.D2, 1.B10 and 3.B4 results in a dose dependent decrease in cell density of SKBR-3 tumor cells, as compared to a control human antibody. The F(ab')₂ fragments of the 3.F2 and 2.E8 anti-HER2/neu monoclonal antibodies also inhibit the growth of SKBR-3 tumor cells in a dose dependent fashion (see Figure 6). Furthermore, as shown in Figure 7A, anti-HER2/neu antibodies 3.F2, 2.E8, 1.D2, 1.B10 and 3.B4 exhibited a dose dependent reduction in the cell density of BT-474 tumor cells, as compared to a control human antibody. Figure 7B shows that treatment of BT-474 tumor cells with 10 µg of the anti-HER2/neu antibodies 3.F2 and 2.E8, as well as their F(ab')₂ fragments, inhibits tumor cell growth, as compared to a control antibody. Bispecific molecules including the human anti-HER2/neu antibodies or antibody fragments (e.g., as shown in Figure 13) can be tested for inhibition of tumor cell growth in this same manner.

### Antibody dependent cell-mediated cytotoxicity (ADCC) activity of human anti-HER2/neu antibodies

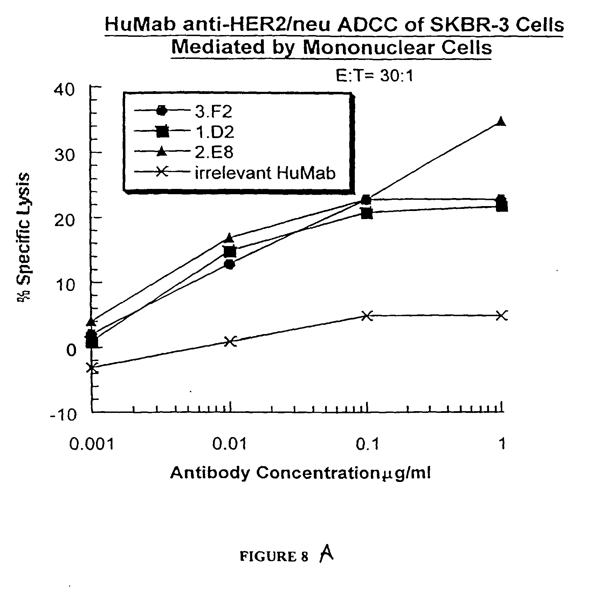

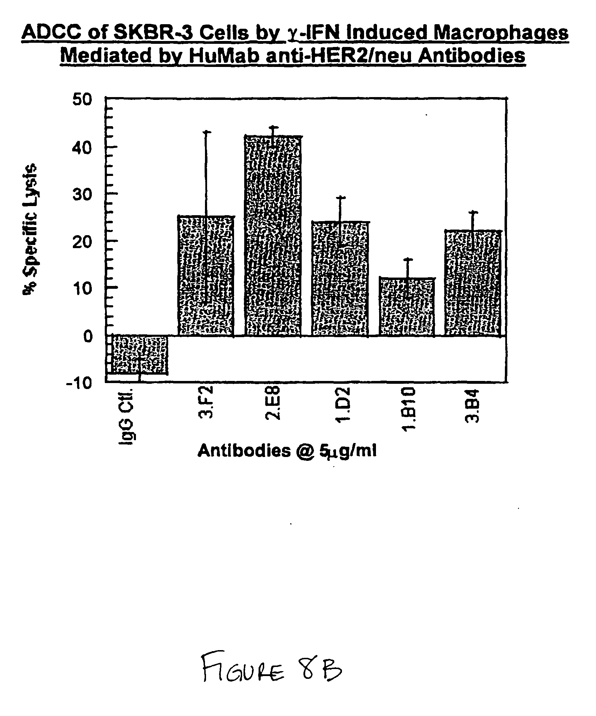

The ability of selected human anti-HER2/neu monoclonal antibodies to mediate ADCC of target tumor cells expressing cell surface HER2/neu *in vitro* was also tested. Briefly, mononuclear cells were isolated from healthy donors and incubated with ⁵¹Cr labeled SKBR-3 tumor cells in the presence of purified anti-HER2/neu antibodies 3.F2, 2.E8 and 1.D2. After 4 hours, the culture supernatant from the wells was harvested and ⁵¹Cr release was measured on a gamma counter. The percent specific lysis was determined according the following formula: (experimental CPM - target leak CPM)/(detergent lysis CPM - target leak CPM) × 100%. Figure 8A demonstrates that the anti-HER2/neu antibodies mediated the dose dependent lysis of SKBR-3 tumor cells as compared to a control human antibody. In another study, IFN-γ induced macrophages were incubated with ⁵¹Cr labeled SKBR-3 cells in the presence of 5 µg/ml of the anti-HER2/neu antibodies 3.F2, 2.E8, 1.D2, 1.B10 and 3.B4. After 4 hours, the culture supernatant was harvested and ⁵¹Cr release was measured as described above. As shown in Figure 8B, the anti-HER2/neu antibodies mediated the dose dependent lysis of HER2/neu overexpressing tumor cells as compared to a control human antibody.

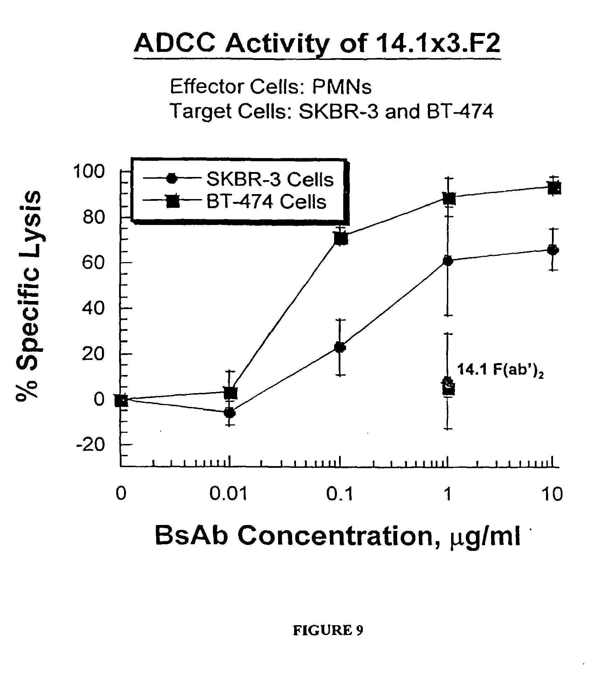

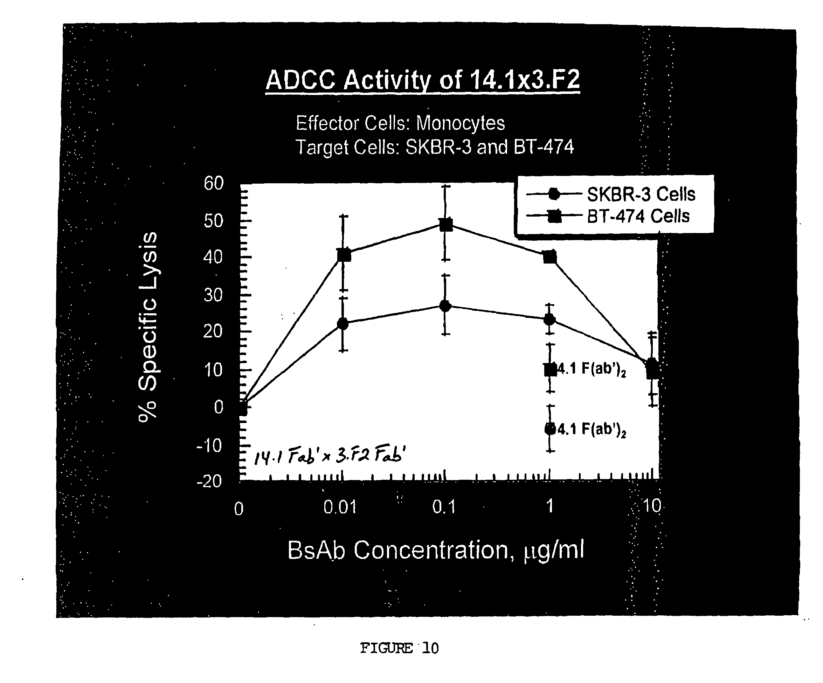

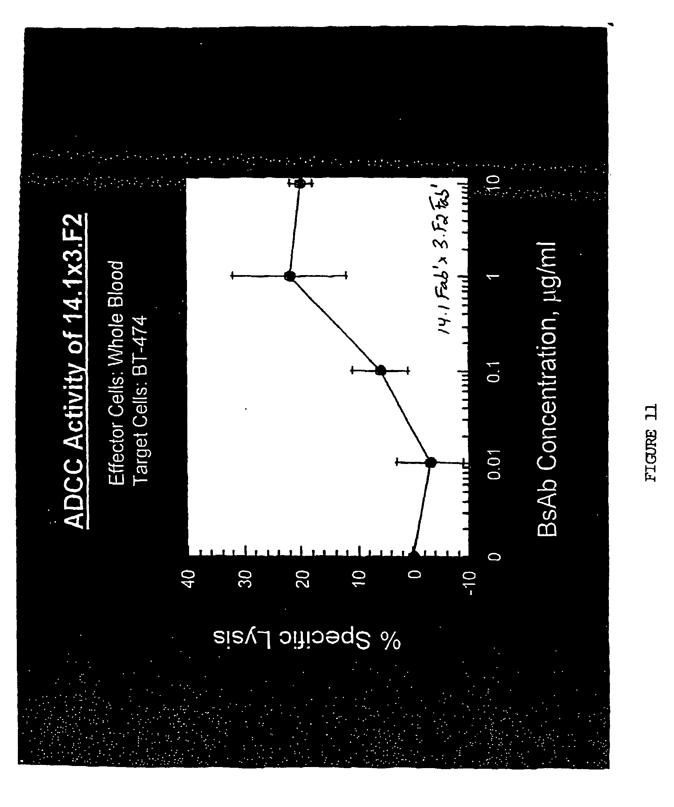

In addition, purified bispecific molecule 14.1 × 3.F2 (Figure 13) was tested for ADCC killing with polymorphonuclear cells of ⁵¹Cr labeled SKBR-3 or BT-474 human tumor cells. After overnight incubation at 37 °C, the amount of tumor cell killing was determined by analysis of ⁵¹Cr released into the culture supernatants. The specific lysis reported is the amount of tumor cell lysis in the presence of the bispecific molecule - tumor cell lysis with polymorphonuclear cells alone. As shown in Figure 9, the bispecific molecule 14.1 × 3.F2 mediated cell killing by PMNs of both SKBR-3 and BT-474 tumor cells expressing HER2/neu in a dose dependent fashion. In addition, as shown in Figure 10, bispecific molecule 14.1 × 3.F2 mediated cell killing by monocytes of both SKBR-3 and BT-474 tumor cells expressing HER2/neu in a dose dependent fashion. In both cases, addition of 10 µg/ml of 14.1 Fab'₂ completely blocked ADCC of the tumor cells by 1 µg/ml of the bispecific molecule 14.1 × 3.F2, demonstrating that targeted cell killing was mediated exclusively by CD89 binding to the effector cells. Figure 11 shows that bispecific molecule 14.1 × 3.F2 also mediated cell killing by whole blood of BT-474 tumor cells expressing HER2/neu in a dose dependent fashion.

The foregoing Examples describe the generation of human monoclonal antibodies and bispecifics that specifically react with high affinity to HER2/neu. In addition, these examples demonstrate that the human monoclonal anti-HER2/neu antibodies effectively inhibit the growth of human tumor cells expressing HER2/neu. In addition, the human anti-HER2/neu antibodies and bispecific molecules mediate cell killing activity in the presence of effector cells against human tumor cells expressing high levels of HER2/neu. These results support the conclusion that the fully human monoclonal antibodies against HER2/neu (as well as fragments thereof) and bispecific molecules including these antibodies of the present invention are useful for the treatment of HER2/neu related disorders.

### Example 5 Bispecific and Trispecific Molecules Containing Human Antibody Binding Portions Directed to HER2/neu and CD89 (FcαR) Promote Cell Mediated Cytotoxicity of Tumor Cells

The following studies involve the production and characterization of bispecific and trispecific molecules which induce effector cell mediated killing of tumor cells expressing EGF Receptor (EGF-R) and/or HER2/neu (also referred to as HER2). The bispecific constructs include an anti-CD89 (anti-FcαR) antibody (ScFv or Fab') linked to an anti-HER2 antibody (ScFv or Fab'). The trispecific construct included the bispecific anti-CD89 (ScFv) x anti-HER2 (ScFv) construct, fused to EGF. Therefore, the constructs activate the effector functions of CD89 expressing cells that have been targeted to tumor cells.

### Generation of Fusion Constructs

Figures 12 and 13 show each of the bi- and tri-specific molecules and their parent human antibodies (HuMabs). Figure 12 shows the single-chain (ScFv) bispecific fusion molecules, 931 and 934, which are the same except that 934 includes EGF, whereas 931 does not. The variable light and heavy chain regions of 14.1 (anti-CD89) and 3F2 (anti-HER2) were used to generate the constructs. Figure 13 shows a chemically conjugated Fab' anti-CD89 x anti-HER2 bispecific molecule (as described in Example 4 above) used as positive control. The fab' fragments of 14.1 and 3F2 were used to generate this BsAb.

The single chain bi- and tri-specific molecules (931 and 934) were produced by transfecting NSO cells with plasmids (e.g., pJZ934) and selected in media containing G418. Cell lines were screened for the expression of fusion protein and subcloned by limited dilution cloning. Finally, fusion protein was purified by running supernatant of cell lines expressing fusion protein over a protein L column and then loading approximately 2 µg of purified protein onto a 4-15% tris-glycine gel under non-reducing and reducing conditions. The 934 construct purified predominantly as a trimer species, which dissociated to monomers under reducing conditions.

### Characterization of Bi- and Tri-Specific Molecule Binding to Tumor Cells

Flow Cytometry was used to characterize binding of the bi- and tri-specific molecules. Figure 14(A) shows binding to tumor cells of fusion proteins in transfectoma supernatants. Transfected (931 & 934) and untransfected (NSO) supernatant was incubated with either SKBR-3 or A431 tumor cell lines. Binding of fusion protein was then detected by staining with goat anti-human IgG fab-2 conjugated to phycoerythrin. As shown in Figure 14(B), supernatants from transfected cells (931 and 934) also mediated cell lysis (ADCC). In these studies, chromium release assays were done with an incubation period of 16-18 h and an effector (monocytes, PMN) to target (SKBR-3, A431) ration of 100:1. Tumor cell killing was detected using transfected (931 & 934) and untransfected (NSO) supernatant to mediate specific lysis.

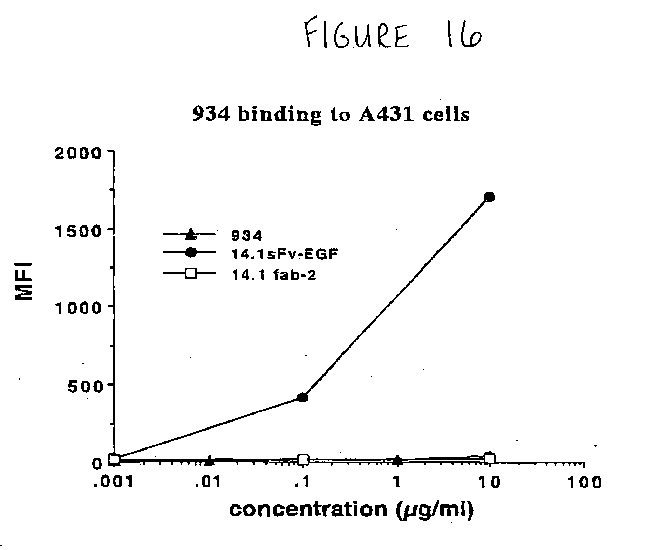

Figure 15(A) shows binding activity of purified bi- and tri-specific molecules (931 and 934) to U937 cells (through the CD89 receptor). The fab-2 fragment of antibody 425 (anti-EGF-R mAb), which does not bind U937 cells, was used as a negative control. The anti-CD89 (Fab') x anti-HER2 (Fab') chemically coupled bispecific molecule (shown in Figure 13) was used as a positive control. Figure 15(B) is the same as Figure 15(A), except that binding to SKBR-3 cells (through the HER2 receptor) was tested. The fab-2 fragment of anti-CD89 antibody, 14.1, was used as a negative control. Figure 16 shows binding of the 934 trispecific construct to A431 cells. This experiment was done the same way as shown in Figure 15, except A431 tumor cells which over-express EGF-R were used. A fusion protein consisting of the sFv fragment of 14.1 fused to EGF was used as a positive control, and the fab-2 fragment of anti-CD89 antibody, 14.1, was used as a negative control.

### Effector Cell-Mediated Lysis of HER2/neu and EGF-R Expressing Tumor Cells

Single chain bi- and tri-specific molecules (931 and 934) also were tested for their ability to mediate cell lysis (ADCC) of Her2/neu and EGF-R expressing tumor cells in the presence of effector cells. Chromium release assays were done with an incubation period of 16-18 h and an effector (monocytes, PMN) to target (SKBR-3, A431) ration of 100:1. Tumor cell killing was detected using transfected (931 & 934) and untransfected (NSO) supernatant to mediate specific lysis. As shown in Figure 14(B), supernatants from transfected cells (931 and 934) mediated cell lysis (ADCC) of monocytes, PMNs and whole blood. In these studies, chromium release assays were done with an incubation period of 16-18 h and an effector (monocytes, PMN) to target (SKBR-3, A431) ration of 100:1. Tumor cell killing was detected using transfected (931 & 934) and untransfected (NSO) supernatant to mediate specific lysis.

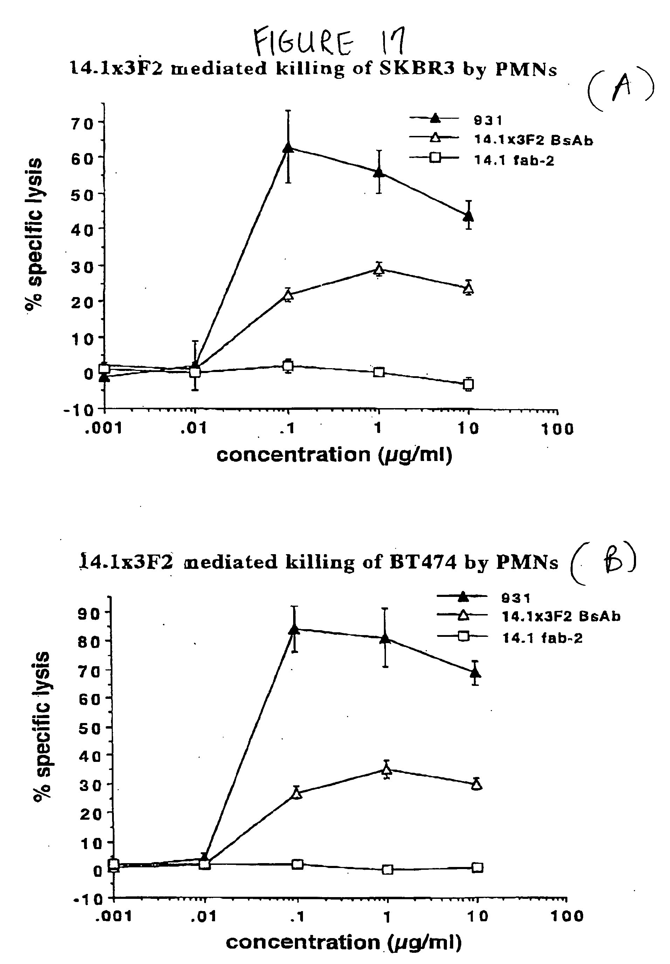

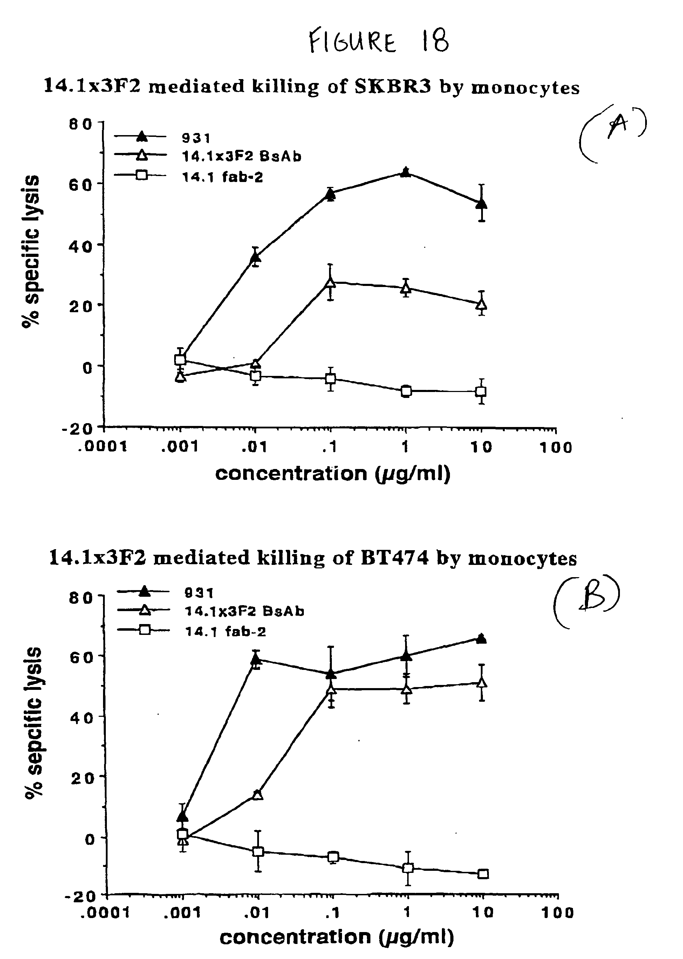

Figure 17 and Figure 18 show effector cell-mediated lysis of SKBR-3 cells (Figure 17(A) and Figure 18(A)) and BT474 cells (Figure 17(B) and Figure 18(B)) by purified single chain bispecific construct, 931, in the presence of PMNs and monocytes, respectively. Chromium release assays were done with an incubation period of 16-18 h and an effector to target ration of 100:1. The fab-2 fragment of anti-CD89 antibody, 14.1, was used as a negative control in each experiment.

Overall, the foregoing studies describe the generation of single chain bi- and tri-specific molecules which target and induce effector cell-mediated killing of tumor cells. The foregoing studies also demonstrate that these bi- and tri-specific molecules are capable of binding to CD89, EGF-R and HER2 expressing cells. Importantly, the studies further demonstrate that, in the presence of CD89-expressing immune effector cells, these bi- and tri-specific molecules mediate killing of EGF-R and HER2 overexpressing cells in a dose-dependent manner, and that cell killing is mediated by binding to CD89 on effector cells.

### References

1. Devilee, P. et al. (1994) Recent developments in the molecular genetic understanding of breast cancer. Critical Reviews Oncogenesis 5:247-270.
2. Stancovski, I. et al. (1991) Mechanistic aspects of the opposing effects of monoclonal antibodies to the ERBB2 receptor on tumor growth. Proc. Natl. Acad. Sci. USA 88:8691-8695.
3. Bargmann, C.I. et al. (1986) Multiple independent activation of the neu oncogene by a point mutation altering the transmembrane domain of p185. Cell 45:649-657.
4. Weiner, D.B. et al. (1989) A point mutation in the neu oncogene mimics ligand induction of receptor aggregation. Nature 339:230-231.
5. Stern, D.F. et al. (1988) Oncogenic activation of p185neu stimulates tyrosine phosphorylation in vivo. Mol. Cell. Biol. 8:3969-3973.
6. Ross, J.S. et al. (1998) The HER2/neu oncogene in breast cancer: prognostic factor, predictive factor, and target for therapy. Stem Cells 16:413-428.
7. Kern, J.A. et al. (1993) Inhibition of human lung cancer cell line growth by an anti-p185HER2 antibody. Am. J. Respir. Cell Mol. Biol. 9:448-454.
8. Witters, L.M. et al. (1997) Enhanced anti-proliferative activity of the combination of tamoxifen plus HER-2-neu antibody. Breast Cancer Res. Treatment 42:1-5.
9. Myers, J.N et al. (1991) Biological effects of monoclonal antireceptor antibodies reactive with neu oncogene product, p185neu. Methods Enzymol. 198: 277-290.
10. Fendly, B.M. et al. (1990) Characterization of murine monoclonal antibodies reactive to either the human epidermal growth factor receptor or Her2/neu gene product. Cancer Res. 50:1550-1558.
11. G.Kohler and Milstein C. (1975) Continuous cultures of fused cells secreting antibody of predefined specificity. Nature 256: 495-497.
12. G.L. Boulianne. Hozum N., and Shulman M.J. (1984) Production of functional chimeric mouse/human antibody. Nature 312: 643-646.
13. P.T. Jones. Dear P.H., Foote J., Neuberger M.S., and Winter G. (1989) Replacing the complementarity-determining regions in a human antibody with those from a mouse. Nature 321: 522-525.
14. J.D. Marks et al. (1991) By-passing Immunization Human antibodies from V-gene libraries displayed on phage. J. Mol. Biol. 222: 581-597.
15. N. Lonberg, et al. 1994. Antigen-specific human antibodies from mice comprising four distinct genetic modifications. Nature 368(6474): 856-859.
16. G.Gafie, Howe S.C., Butcher M.C. C. W., and Howard H.C. (1997) Antibodies to major histocompatibility antigens produced by hybrid cell lines. Nature 266:550-552.

## Claims

1. An isolated human monoclonal antibody or antibody fragment that binds to HER2/neu comprising a human heavy chain variable region and human light chain variable region wherein:
a) the human heavy chain variable region comprises an amino acid sequence selected from the group consisting of SEQ ID NO: 2 and sequences that have at least 90% identity to SEQ ID NO: 2; and
b) the human light chain variable region comprises an amino acid sequence selected from the group consisting of SEQ ID NO: 4 and sequences that have at least 90% identity to SEQ ID NO: 4.

2. The human antibody or antibody fragment of claim 1. wherein the antibody has the ability to mediate cytolysis of a cell expressing HER2/neu in the presence of human effector cells at an IC50 of 1 x 10⁻⁷ M or less *in vitro.*

3. The human antibody or antibody fragment of claim 1 or 2 which is capable of inhibiting growth or mediating cytolysis of a cell expressing HER2/neu at a concentration of about 10 µg/ml or less *in vitro* in the presence of human effector cells.

4. The human antibody or antibody fragment of any of the preceding claims having the ability to be internalized by HER2/neu expressing cells after binding to HER2/neu.

5. The human antibody or antibody fragment of any of the preceding claims having the ability to opsonize a cell expressing HER2/neu.

6. The human antibody or antibody fragment of any one of the preceding claims having the ability to bind human HER2/neu with an affinity constant (K_{A}) of at least 10⁷ M⁻¹.

7. The human antibody or antibody fragment of any one of the preceding claims having the ability to bind to human HER2/neu with an affinity constant (K_{Λ}) of at least 10⁸ M⁻¹.

8. The human antibody or antibody fragment of any one of the preceding claims having the ability to bind to human HER2/neu with an affinity constant (K_{Λ}) of at least 10⁹ M⁻¹.

9. The human antibody or antibody fragment of any one of the preceding claims having the ability to dissociate from human HER2/neu with a rate constant (K_{dis}) of about 10⁻³ S⁻¹ or less.

10. The human antibody or antibody fragment of any one of the preceding claims having an isotype selected from the group consisting of IgG 1, IgG2, IgG3, IgG4, IgM. IgA 1, IgA2, IgAsec, IgD, and IgE.

11. The human antibody or antibody fragment of any one of the preceding claims which is an IgG1κ.

12. The human antibody or antibody fragment of any of the preceding claims, wherein the cell expressing HER2/neu is a tumor cell.

13. The human antibody or antibody fragment of claim 12, wherein the cell expressing HER2/neu is selected from the group consisting of an adenocarcinoma cell, e.g. salivary gland, stomach and kidney, a mammary gland carcinoma cell, a lung carcinoma cell, a squamous cell carcinoma cell, and an ovarian cancer cell.

14. The human antibody or antibody fragment of any one of the preceding claims having the ability to inhibit tumor cell growth by at least about 40%.

15. The human antibody or antibody fragment of any one of the preceding claims having the ability to inhibit tumor cell growth by at least about 60%.

16. The human antibody or antibody fragment of any one of the preceding claims, wherein the antibody or antibody fragment is produced from a transgenic non-human animal.

17. A human antibody or antibody fragment of any preceding claim for use in medicine.

18. A composition comprising the human antibody or antibody fragment of any one of claims 1-16 and a pharmaceutically acceptable carrier.

19. A composition according to claim 18 for use in medicine.

20. An immunoconjugate comprising the human antibody or antibody fragment of any one of claims 1-16 linked to a therapeutic agent.

21. The immunoconjugate of claim 20 wherein the therapeutic agent is a cytotoxin.

22. An immunoconjugate of claim 20 or 21 for use in medicine.

23. A composition comprising the immunoconjugate of any one of claims 20 to 22, and a pharmaceutically acceptable carrier.

24. A composition according to claim 23 for use in medicine.

25. A bispecific molecule comprising the human antibody or antibody fragment of any one of claims 1-16 and further comprising an additional binding specificity for an Fc receptor.

26. The bispecific molecule of claim 25, wherein the Fc receptor is a human Fcγ receptor or a human Fcα receptor.

27. A bispecific molecule of claim 25 or 26 for use in medicine.

28. In vitro use of an antibody or antibody fragment according to any one of claims 1-16 to inhibit growth of a tumor cell expressing HER2/neu.

29. In vitro use of claim 28, wherein the tumor cell is selected from the group consisting of an adenocarcinoma cell, a salivary gland carcinoma cell, a stomach cancer cell, a kidney cancer cell, a mammary gland carcinoma cell, a lung carcinoma cell, a squamous cell carcinoma cell, and an ovarian cancer cell.

30. In vitro use of an antibody or antibody fragment according to any one of claims 1-16 to induce cytolysis of cell expressing HER2/neu by an effector cell.

31. Use of an antibody or antibody fragment according to any one of claims 1-16 in the manufacture of a medicament for inhibiting growth of a tumor cell expressing HER2/neu.

32. The use of claim 31, wherein the tumor cell is selected from the group consisting of an adenocarcinoma cell, a salivary gland carcinoma cell, a stomach cancer cell, a kidney cancer cell, a mammary gland carcinoma cell, a lung carcinoma cell, a squamous cell carcinoma cell, and an ovarian cancer cell.

33. Use of an antibody or antibody fragment according to any one of claims 1-16 in the manufacture of a medicament for preventing a cancer selected from the group consisting of adenocarcinoma, *e.g.,* salivary gland, stomach and kidney carcinoma, mammary gland carcinoma, lung carcinoma, squamous cell carcinoma, and ovarian cancer, for example by inducing cytolysis of a cell expressing HER2/neu by a effector cell.

## Patentansprüche

1. Isolierter humaner monoklonaler Antikörper oder isoliertes humanes Antikörperfragment, das an Her2/neu bindet, umfassend, einen variablem Bereich der humanen schweren Kette und einen variablen Bereich der humanen leichten Kette, worin:
a) der variable Bereich der humanen schweren Kette eine Aminosäuresequenz umfaßt, ausgewählt aus der Gruppe bestehend aus SEQ. ID. Nr.2 und Sequenzen, die mindestens 90 % Identität zur SEQ. ID. Nr.2 aufweisen; und
b) der variable Bereich der humanen leichten Kette eine Aminosäuresequenz umfaßt, ausgewählt aus der Gruppe bestehend aus Seq. ID. Nr.4 und Sequenzen, die mindestens 90 % Identität zur Seq. ID. Nr.4 aufweisen.

2. Humaner Antikörper oder Antikörperfragment nach Anspruch 1, worin der Antikörper die Zytolyse einer HER2/neu exprimierenden Zelle in Anwesenheit von humanen Effektorzellen bei einem IC50 von 1 x 10-7 M oder weniger *in vitro* vermitteln kann.

3. Humaner Antikörper oder Antikörperfragment nach Anspruch 1 oder 2, der/das das Wachstum einer HER/neu exprimierenden Zelle bei einer Konzentration von 10 µg/ml oder weniger *in vitro* inhibieren oder in der Anwesenheit von humanen Effektorzellen deren Zytolyse vermitteln kann.

4. Humaner Antikörper oder Antikörperfragment nach einem der vorhergehenden Ansprüche, der in HER2/neu exprimierende Zellen nach Binden an Her2/neu internalisiert werden kann.

5. Humaner Antikörper oder Antikörperfragment nach einem der vorhergehenden Ansprüche, der eine Her2/neu exprimierende Zelle opsonieren kann.

6. Humaner Antikörper oder Antikörperfragment nach einem der vorhergehenden Ansprüche, der HER2/neu mit einer Affinitätskonstante (K_{Λ}) von mindestens 10⁷ M⁻¹ binden kann.

7. Humaner Antikörper oder Antikörperfragment nach einem der vorhergehenden Ansprüche, der humanes HER2/neu mit einer Affinitätskonstante (K_{Λ}) von mindestens 10⁸ M⁻¹ binden kann.

8. Humaner Antikörper oder Antikörperfragment nach einem der vorhergehenden Ansprüche, der humanes HER2/neu mit einer Affinitätskonstante (K_{Λ}) von mindestens 10⁹ M⁻¹ binden kann.

9. Humaner Antikörper oder Antikörperfragment nach einem der vorhergehenden Ansprüche, der von humanem HER2/neu mit einer konstanten Rate (K_{diss}) von etwa 10⁻³S⁻¹ oder weniger dissoziieren kann.

10. Humaner Antikörper oder Antikörperfragment nach einem der vorhergehenden Ansprüche, der einen Isotyp aufweist ausgewählt aus der Gruppe bestehend aus IgG1, IgG2, IgG4, IgM, IgA1, IgA2, IgAsec, IgD und IgE.

11. Humaner Antikörper oder Antikörperfragment nach einem der vorhergehenden Ansprüche, der ein IgG1κ ist.

12. Humaner Antikörper oder Antikörperfragment nach einem der vorhergehenden Ansprüche, worin die HER2/neu exprimierende Zelle eine Tumorzelle ist.

13. Humaner Antikörper oder Antikörperfragment nach Anspruch 12, worin die HER2/neu exprimierende Zelle ausgewählt ist aus der Gruppe bestehend aus einer Adenokarzinomzelle, beispielsweise der Speicheldrüse, des Magens und der Niere, einer Brustdrüsenkkarzinomzelle, einer Lungenkarzinomzelle, einer Plattenepithelzellen-Karzinomzelle, und einer Eierstock-Krebszelle.

14. Humaner Antikörper oder Antikörperfragment nach einem der vorhergehenden Ansprüche, der die Fähigkeit hat, Tumorzellwachstum um mindestens etwa 40 % zu inhibieren.

15. Humaner Antikörper oder Antikörperfragment nach einem der vorhergehenden Ansprüche, der die Fähigkeit hat, Tumorzellwachstum um mindestens etwa 60 % zu inhibieren.

16. Humaner Antikörper oder Antikörperfragment nach einem der vorhergehenden Ansprüche, worin der Antikörper oder das Antikörperfragment von einem transgenen, nichtmenschlichen Tier hergestellt wird.

17. Humaner Antikörper oder Antikörperfragment nach einem der vorhergehenden Ansprüche zur Verwendung in der Medizin.

18. Zusammensetzung, welche den humanen Antikörper oder Antikörperfragment nach einem der Ansprüche 1-16 und einen pharmazeutisch verträglichen Träger umfaßt.

19. Zusammensetzung nach Anspruch 18 zur Verwendung in der Medizin.

20. Immunkonjugat, welches den humanen Antikörper oder Antikörperfragment nach einem der Ansprüche 1 - 16, gebunden an ein therapeutisches Mittel, umfaßt.

21. Immunkonjugat nach Anspruch 20, worin das therapeutische Mittel ein Zytotoxin ist.

22. Immunkonjugat nach Anspruch 20 oder 21 zur Verwendung in der Medizin.

23. Zusammensetzung, welche ein Immunkonjugat nach einem der Ansprüche 20 bis 22 und einen pharmazeutisch annehmbaren Träger umfaßt.

24. Zusammensetzung nach Anspruch 23 zur Verwendung in der Medizin.

25. Bispezifisches Molekül, welches den humanen Antikörper oder Antikörperfragment nach einem der Ansprüche 1-16 und weiter eine zusätzliche Bindungsspezifität für einen Fc-Rezeptor umfaßt.

26. Bispezifisches Molekül nach Anspruch 25, worin der Fc-Rezeptor ein humaner Fcγ-Rezeptor oder ein humaner Fcα-Rezeptor ist.

27. Bispezifisches Molekül nach Anspruch 25 oder 26 zur Verwendung in der Medizin.

28. In vitro Verwendung eines Antikörpers oder Antikörperfragments nach einem der Ansprüche 1 - 16, um das Wachstum einer HER2/neu exprimierenden Zelle zu inhibieren.

29. In vitro Verwendung nach Anspruch 28, worin die Tumorzelle ausgewählt ist aus der Gruppe bestehend einer Adenokarzinomzelle, einer Speicheldrüsenkarzinomzelle, einer Magen-Krebszelle, einer Nieren-Krebszelle, einer Brustdrüsenkarzinomzelle, einer Lungenkarzinomzelle, einer Plattenepithelzellen-Karzinomzelle, und einer Eierstock-Krebszelle.

30. In vitro Verwendung eines Antikörpers oder Antikörperfragments nach einem der Ansprüche 1 - 16, um die Zytolyse einer HER2/neu exprimierenden Zelle mittels einer Effektorzelle zu induzieren.

31. Verwendung eines Antikörpers oder Antikörperfragments nach einem der Ansprüche 1 - 16 zur Herstellung eines Medikaments, um das Wachstum einer HER2/neu exprimierenden Zelle zu inhibieren.

32. Verwendung nach Anspruch 31, worin die Tumorzelle ausgewählt ist aus der Gruppe bestehend aus einer Adenokarzinomzelle, einer Speicheldrüsenkarzinomzelle, einer Magen-Krebszelle, einer Nieren-Krebszelle, einer Brustdrüsenkarzinomzelle, einer Lungenkarzinomzelle, einer Plattenepithelzellen-Karzinomzelle, und einer Eierstock-Krebszelle.

33. Verwendung eines Antikörpers oder Antikörperfragments nach einem der Ansprüche 1 - 16 zur Herstellung eines Medikaments, um Krebs zu verhindern ausgewählt aus der Gruppe bestehend aus Adenokarzinom, beispielsweise Speicheldrüsen-, Magen- und Nierenkarzinom, Brustdrüsenkarzinom, Lungenkarzinom, Plattenepithelzellen-Karzinom, und Eierstock-Krebs, indem beispielsweise die Zytolyse einer HER2/neu exprimierenden Zelle mittels einer Effektorzelle induziert wird.

## Revendications

1. Anticorps ou fragment d'anticorps monoclonal humain isolé qui se lie à HER2/neu comprenant une région variable de chaîne lourde humaine et une région variable de chaîne légère humaine, dans lequel :
a) la région variable de chaîne lourde humaine comprend une séquence d'acides aminés choisie parmi le groupe constitué par SEQ ID NO: 2 et des séquences qui possèdent une identité à concurrence d'au moins 90 % à SEQ ID NO: 2 ; et
b) la région variable de chaîne légère humaine comprend une séquence d'acides aminés choisie parmi le groupe constitué par SEQ ID NO: 4 et des séquences qui possèdent une identité à concurrence d'au moins 90 % à SEQ ID NO: 4.

2. Anticorps ou fragment d'anticorps humain selon la revendication 1, dans lequel l'anticorps a la capacité de faciliter la cytolyse d'une cellule exprimant HER2/neu en présence de cellules effectrices humaines à une valeur CI₅₀ de 1 x 10⁻⁷ M ou moins *in vitro.*

3. Anticorps ou fragment d'anticorps humain selon la revendication 1 ou 2, qui est capable d'inhiber la croissance ou de faciliter la cytolyse d'une cellule exprimant HER2/neu à une concentration d'environ 10 µg/ml ou moins in vitro en présence de cellules effectrices humaines.

4. Anticorps ou fragment d'anticorps humain selon l'une quelconque des revendications précédentes, ayant la capacité d'être internalisé par des cellules exprimant HER2/neu après une liaison au HER2/neu.

5. Anticorps ou fragment d'anticorps humain selon l'une quelconque des revendications précédentes, ayant la capacité d'opsoniser une cellule exprimant HER2/neu.

6. Anticorps ou fragment d'anticorps humain selon l'une quelconque des revendications précédentes, ayant la capacité de se lier au récepteur humain HER2/neu avec une constante d'affinité (K_{A}) d'au moins 10⁷ M⁻¹.

7. Anticorps ou fragment d'anticorps humain selon l'une quelconque des revendications précédentes, ayant la capacité de se lier au récepteur humain HER2/neu avec une constante d'affinité (K_{A}) d'au moins 10⁸ M⁻¹.

8. Anticorps ou fragment d'anticorps humain selon l'une quelconque des revendications précédentes, ayant la capacité de se lier au récepteur humain HER2/neu avec une constante d'affinité (K_{A}) d'au moins 10⁹ M⁻¹.

9. Anticorps ou fragment d'anticorps humain selon l'une quelconque des revendications précédentes, ayant la capacité de se dissocier du récepteur humain HER2/neu avec une constante de vitesse (K_{DIS}) d'environ 10⁻³ S⁻¹ ou moins.

10. Anticorps ou fragment d'anticorps humain selon l'une quelconque des revendications précédentes, possédant un isotype choisi parmi le groupe constitué par IgG1, IgG2, IgG3, IgG4, IgM, IgA1, IgA2, IgAsec, IgD et IgGE.

11. Anticorps ou fragment d'anticorps humain selon l'une quelconque des revendications précédentes, un savoir une IgIκ.

12. Anticorps ou fragment d'anticorps humain selon l'une quelconque des revendications précédentes, la cellule exprimant HER2/neu est une cellule tumorale.

13. Anticorps ou fragment d'anticorps humain selon la revendication 12, dans lequel la cellule exprimant HER2/neu est choisie parmi le groupe constitué par une cellule d'adénocarcinome, par exemple d'une glande salivaire, de l'estomac et des reins, une cellule de carcinome de glande mammaire, une cellule de carcinome de poumon, une cellule de carcinome de cellule squameuse et une cellule de cancer ovarien.

14. Anticorps ou fragment d'anticorps humain selon l'une quelconque des revendications précédentes, ayant la capacité d'inhiber une croissance de cellules tumorales à concurrence d'au moins environ 40 %.

15. Anticorps ou fragment d'anticorps humain selon l'une quelconque des revendications précédentes, ayant la capacité d'inhiber une croissance de cellules tumorales à concurrence d'au moins environ 60 %.

16. Anticorps ou fragment d'anticorps humain selon l'une quelconque des revendications précédentes, dans lequel l'anticorps ou le fragment d'anticorps est produit à partir d'un animal transgénique non humain.

17. Anticorps ou fragment d'anticorps humain selon l'une quelconque des revendications précédentes, à utiliser en médecine.

18. Composition comprenant l'anticorps ou le fragment d'anticorps humain selon l'une quelconque des revendications 1 à 16 et un support pharmaceutiquement acceptable.

19. Composition selon la revendication 18, à utiliser en médecine.

20. Immunoconjugué comprenant l'anticorps ou le fragment d'anticorps humain selon la revendication 16, lié à un agent thérapeutique.

21. Immunoconjugué selon la revendication 20, dans lequel l'agent thérapeutique est une cytotoxine.

22. Immunoconjugué selon la revendication 20 ou 21, à utiliser en médecine.

23. Composition comprenant l'immunoconjugué selon l'une quelconque des revendications 20 à 22, et un support pharmaceutiquement acceptable.

24. Composition selon la revendication 23, à utiliser en médecine.

25. Molécule bispécifique comprenant l'anticorps ou le fragment d'anticorps humain selon l'une quelconque des revendications 1 à 16, et comprenant en outre une spécificité de liaison supplémentaire pour un récepteur Fc.

26. Molécule bispécifique selon la revendication 25, dans lequel le récepteur Fc est un récepteur Fcγ humain ou un récepteur Fcα humain.

27. Molécule bispécifique selon la revendication 25 ou 26, à utiliser en médecine.

28. Utilisation in vitro d'un anticorps ou d'un fragment d'anticorps selon l'une quelconque des revendications 1 à 16, pour inhiber la croissance d'une cellule tumorale exprimant HER2/neu.

29. Utilisation in vitro selon la revendication 28, dans laquelle la cellule tumorale est choisie parmi le groupe constitué par une cellule d'adénocarcinome, une cellule de carcinome d'une glande salivaire, une cellule de carcinome de l'estomac, une cellule de carcinome des reins, une cellule de carcinome de glande mammaire, une cellule de carcinome de poumon, une cellule de carcinome de cellule squameuse et une cellule de cancer ovarien.

30. Utilisation in vitro d'un anticorps ou d'un fragment d'anticorps selon l'une quelconque des revendications 1 à 16, pour induire une cytolyse d'une cellule exprimant HER2/neu via une cellule effectrice.

31. Utilisation d'un anticorps ou d'un fragment d'anticorps selon l'une quelconque des revendications 1 à 16, pour la préparation d'un médicament destiné à inhiber la croissance d'une cellule tumorale exprimant HER2/neu.

32. Utilisation selon la revendication 31, dans laquelle la cellule tumorale est choisie parmi le groupe constitué par une cellule d'adénocarcinome, une cellule de carcinome d'une glande salivaire, une cellule de carcinome de l'estomac, une cellule de carcinome des reins, une cellule de carcinome de glande mammaire, une cellule de carcinome de poumon, une cellule de carcinome de cellule squameuse et une cellule de cancer ovarien.

33. Utilisation d'un anticorps ou d'un fragment d'anticorps selon l'une quelconque des revendications 1 à 16, pour la préparation d'un médicament destiné à prévenir un cancer choisi parmi le groupe constitué par un adénocarcinome, par exemple d'une glande salivaire, de l'estomac et des reins, un carcinome de glande mammaire, un carcinome de poumon, un carcinome de cellule squameuse et un cancer ovarien, par exemple en induisant une cytolyse d'une cellule exprimant HER2/neu via une cellule effectrice.


### SEQUENCE LISTING

<110> Medarex, Inc.
<120> HUMAN MONOCLONAL ANTIBODIES TO HER2/NEU
<130> MXI-160PC
<140>
   <141>
<150> USSN 60/146,313
   <151> 1999-07-29
<150> USSN 60/188,539
   <151> 1999-03-10
<160> 13
<170> PatentIn Ver. 2.0
<210> 1
   <211> 372
   <212> DNA
   <213> Homo sapiens
<220>
   <221> CDS
   <222> (1)..(372)
<400> 1
   gag gtg cag ctg ttg gag tct ggg gga ggc ttg gta cag cct ggg ggg    48
   Glu Val Gln Leu Leu Glu Ser Gly Gly Gly Leu Val Gln Pro Gly Gly
     1               5                  10                  15
   tcc ctg aga ctc tcc tgt gca gcc tct gga ttc acc ttt agc agc tat    96
   Ser Leu Arg Leu Ser Cys Ala Ala Ser Gly Phe Thr Phe Ser Ser Tyr
                20                  25                  30
   gcc atg acc tgg gtc cgc cag gct cca ggg aag ggg ctg gag tgg gtc
   144
   Ala Met Thr Trp Val Arg Gln Ala Pro Gly Lys Gly Leu Glu Trp Val
            35                  40                  45
   tca gct atc agt ggt agt ggt tat agc aca tac tac gca gac tcc gag
   192
   Ser Ala Ile Ser Gly Ser Gly Tyr Ser Thr Tyr Tyr Ala Asp Ser Glu
        50                  55                  60
   aag ggc cgg ttc acc atc tcc aga gac aat tcc aag aac acg ctg tat
   240
   Lys Gly Arg Phe Thr Ile Ser Arg Asp Asn Ser Lys Asn Thr Leu Tyr
    65                  70                  75                  80
   ctg caa atg aac agc ctg aga gcc gag gac acg gcc gta tat tac tgt
   288
   Leu Gln Met Asn Ser Leu Arg Ala Glu Asp Thr Ala Val Tyr Tyr Cys
                   85                  90                  95
   gcg aaa ggg ttt cag tat ggt tcg ggg agt tat tat acc cac ttt gac
   336
   Ala Lys Gly Phe Gln Tyr Gly Ser Gly Ser Tyr Tyr Thr His Phe Asp
               100                 105                 110
   tac tgg ggc cag gga acc ctg gtc acc gtc tcc tca
   372
   Tyr Trp Gly Gln Gly Thr Leu Val Thr Val Ser Ser
           115                 120
<210> 2
   <211> 124
   <212> PRT
   <213> Homo sapiens
<400> 2
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Thr Trp Val Arg Gln Ala Pro Gly Lys Gly Leu Glu Trp Val
           35                  40                  45
   Ser Ala Ile Ser Gly Ser Gly Tyr Ser Thr Tyr Tyr Ala Asp Ser Glu
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Phe Gln Tyr Gly Ser Gly Ser Tyr Tyr Thr His Phe Asp
               100                 105                 110
   Tyr Trp Gly Gln Gly Thr Leu Val Thr Val Ser Ser
           115                 120
<210> 3
   <211> 321
   <212> DNA
   <213> Homo sapiens
<220>
   <221> CDS
   <222> (1)..(321)
<400> 3
   gac atc cag atg acc cag tct cca tcc tca ctg tct gca tct gta gga    48
   Asp Ile Gln Met Thr Gln Ser Pro Ser Ser Leu Ser Ala Ser Val Gly
     1               5                  10                  15
   gac aga gtc acc atc act tgt cgg gcg agt cag ggt att agc agc tgg    96
   Asp Arg Val Thr Ile Thr Cys Arg Ala Ser Gln Gly Ile Ser Ser Trp
                20                  25                  30
   tta gcc tgg tat cag cag aaa cca gag aaa gcc cct aag tcc ctg atc
   144
   Leu Ala Trp Tyr Gln Gln Lys Pro Glu Lys Ala Pro Lys Ser Leu Ile
            35                  40                  45
   tat gct gca tcc agt ttg caa agt ggg gtc cca tca agg ttc agc ggc
   192
   Tyr Ala Ala Ser Ser Leu Gln Ser Gly Val Pro Ser Arg Phe Ser Gly
        50                  55                  60
   agt gga tct ggg aca gat ttc act ctc acc atc agc agc ctg cag cct
   240
   Ser Gly Ser Gly Thr Asp Phe Thr Leu Thr Ile Ser Ser Leu Gln Pro
    65                  70                  75                  80
   gaa gat ttt gca act tat tac tgc caa cag tat aat agt tac ccg tac
   288
   Glu Asp Phe Ala Thr Tyr Tyr Cys Gln Gln Tyr Asn Ser Tyr Pro Tyr
                    85                  90                  95
   act ttt ggc cag ggg acc aag ctg gag atc aaa
   321
   Thr Phe Gly Gln Gly Thr Lys Leu Glu Ile Lys
               100                 105
<210> 4
   <211> 107
   <212> PRT
   <213> Homo sapiens
<400> 4
   Asp Ile Gln Met Thr Gln Ser Pro Ser Ser Leu Ser Ala Ser Val Gly
     1               5                  10                  15
   Asp Arg Val Thr Ile Thr Cys Arg Ala Ser Gln Gly Ile Ser Ser Trp
                20                  25                  30
   Leu Ala Trp Tyr Gln Gln Lys Pro Glu Lys Ala Pro Lys Ser Leu Ile
            35                  40                  45
   Tyr Ala Ala Ser Ser Leu Gln Ser Gly Val Pro Ser Arg Phe Ser Gly
        50                  55                  60
   Ser Gly Ser Gly Thr Asp Phe Thr Leu Thr Ile Ser Ser Leu Gln Pro
    65                  70                  75                  80
   Glu Asp Phe Ala Thr Tyr Tyr Cys Gln Gln Tyr Asn Ser Tyr Pro Tyr
                   85                  90                  95
   Thr Phe Gly Gln Gly Thr Lys Leu Glu Ile Lys
               100                 105
<210> 5
   <211> 366
   <212> DNA
   <213> Homo sapiens
<220>
   <221> CDS
   <222> (1)..(366)
<400> 5
   cag gtg cag ctg gtg gag tct ggg gga ggc gtg gtc cag cct ggg agg    48
   Gln Val Gln Leu Val Glu Ser Gly Gly Gly Val Val Gln Pro Gly Arg
   1               5                   10                  15
   tcc ctg aga ctc tcc tgt gca gcg tct gga ttc acc ttc agt agc tat    96
   Ser Leu Arg Leu Ser Cys Ala Ala Ser Gly Phe Thr Phe Ser Ser Tyr
               20                  25                  30
   gac ata cac tgg gtc cgc cag gct cca ggc aag ggg ctg gag tgg gtg
   144
   Asp Ile His Trp Val Arg Gln Ala Pro Gly Lys Gly Leu Glu Trp Val
           35                  40                  45
   gca gta ata tgg tat gat ggc agt aat aaa tac cat gca gac tcc gtg
   192
   Ala Val Ile Trp Tyr Asp Gly Ser Asn Lys Tyr His Ala Asp Ser Val
       50                  55                  60
   aag ggc cga ttc acc atc tcc aga gac aat tcc aag aac acg ctg tat
   240
   Lys Gly Arg Phe Thr Ile Ser Arg Asp Asn Ser Lys Asn Thr Leu Tyr
   65                  70                  75                  80
   ctg caa atg aac agt ctg aga gcc gag gac acg gct gtg tat tac tgt
   288
   Leu Gln Met Asn Ser Leu Arg Ala Glu Asp Thr Ala Val Tyr Tyr Cys
                   85                  90                  95
   gcg aga aac tat ggt ttg ggg agt tat tat aac tac ttt gac ttc tgg
   336
   Ala Arg Asn Tyr Gly Leu Gly Ser Tyr Tyr Asn Tyr Phe Asp Phe Trp
               100                 105                 110
   ggc cag gga acc ctg gtc acc gtc tcc tca
   366
   Gly Gln Gly Thr Leu Val Thr Val Ser Ser
           115                 120
<210> 6
   <211> 122
   <212> PRT
   <213> Homo sapiens
<400> 6
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Asp Ile His Trp Val Arg Gln Ala Pro Gly Lys Gly Leu Glu Trp Val
           35                  40                  45
   Ala Val Ile Trp Tyr Asp Gly Ser Asn Lys Tyr His Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Tyr Gly Leu Gly Ser Tyr Tyr Asn Tyr Phe Asp Phe Trp
               100                 105                 110
   Gly Gln Gly Thr Leu Val Thr Val Ser Ser
           115                 120
<210> 7
   <211> 321
   <212> DNA
   <213> Homo sapiens
<220>
   <221> CDS
   <222> (1)..(321)
<400> 7
   gac atc cag atg acc cag tct cca tcc tca ctg tct gca tct gta gga    48
   Asp Ile Gln Met Thr Gln Ser Pro Ser Ser Leu Ser Ala Ser Val Gly
     1               5                  10                  15
   gac aga gtc acc atc act tgt cgg gcg agt cat ggt att agc agc tgg    96
   Asp Arg Val Thr Ile Thr Cys Arg Ala Ser His Gly Ile Ser Ser Trp
                20                  25                  30
   tta gcc tgg tat cag cag aaa cca gag aaa gcc cct aag tcc ctg atc
   144
   Leu Ala Trp Tyr Gln Gln Lys Pro Glu Lys Ala Pro Lys Ser Leu Ile
            35                  40                  45
   tat gct gca tcc agt ttg caa agt ggg gtc cca tca agg ttc agc ggc
     192
     Tyr Ala Ala Ser Ser Leu Gln Ser Gly Val Pro Ser Arg Phe Ser Gly
          50                  55                  60
   agt gga tct ggg aca gat ttc act ctc acc atc agc agc ctg cag cct
   240
   Ser Gly Ser Gly Thr Asp Phe Thr Leu Thr Ile Ser Ser Leu Gln Pro
    65                  70                  75                  80
   gaa gat ttt gca act tat tac tgc caa cag tat aat agt tac ccg tac
   288
   Glu Asp Phe Ala Thr Tyr Tyr Cys Gln Gln Tyr Asn Ser Tyr Pro Tyr
                    85                  90                  95
   act ttt ggc cag ggg acc aag ctg gag atc aaa
   321
   Thr Phe Gly Gln Gly Thr Lys Leu Glu Ile Lys
               100                 105
<210> 8
   <211> 107
   <212> PRT
   <213> Homo sapiens
<400> 8
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser His Gly Ile Ser Ser Trp
               20                  25                  30
   Leu Ala Trp Tyr Gln Gln Lys Pro Glu Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Pro Tyr
                   85                  90                  95
   Thr Phe Gly Gln Gly Thr Lys Leu Glu Ile Lys
               100                 105
<210> 9
   <211> 366
   <212> DNA
   <213> Homo sapiens
<220>
   <221> CDS
   <222> (1)..(366)
<400> 9
   cag gtg cag ctg gtg gag tct ggg gga ggc gtg gtc cag cct ggg agg    48
   Gln Val Gln Leu Val Glu Ser Gly Gly Gly Val Val Gln Pro Gly Arg
    1               5                  10                  15
   tcc ctg aga ctc tcc tgt gca gcg tct gga ttc acc ttc agt agc tat    96
   Ser Leu Arg Leu Ser Cys Ala Ala Ser Gly Phe Thr Phe Ser Ser Tyr
               20                  25                  30
   gtc atg cac tgg gtc cgc cag gct cca ggc aag ggg ctg gag tgg gtg
   144
   Val Met His Trp Val Arg Gln Ala Pro Gly Lys Gly Leu Glu Trp Val
           35                  40                  45
   gca gtt ata tgg tat gat gga agt aat aaa tac tat gca gac tcc gtg
   192
   Ala Val Ile Trp Tyr Asp Gly Ser Asn Lys Tyr Tyr Ala Asp Ser Val
       50                  55                  60
   aag ggc cga ttc acc atc tcc aga gac aat tcc aag aac acg ctg tat
   240
   Lys Gly Arg Phe Thr Ile Ser Arg Asp Asn Ser Lys Asn Thr Leu Tyr
    65                 70                  75                  80
   ctg caa atg aac agc ctg aga gcc gag gac acg gct gtg tat tac tgt
   288
   Leu Gln Met Asn Ser Leu Arg Ala Glu Asp Thr Ala Val Tyr Tyr Cys
                   85                  90                  95
   gcg ctt atg gtt cgg gga ctt att ata acg ggg tac ttt gac tac tgg
   336
   Ala Leu Met Val Arg Gly Leu Ile Ile Thr Gly Tyr Phe Asp Tyr Trp
               100                 105                 110
   ggc cag gga acc ctg gtc acc gtc tcc tca
   366
   Gly Gln Gly Thr Leu Val Thr Val Ser Ser
           115                 120
<210> 10
   <211> 122
   <212> PRT
   <213> Homo sapiens
<400> 10
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Val Met His Trp Val Arg Gln Ala Pro Gly Lys Gly Leu Glu Trp Val
           35                  40                  45
   Ala Val Ile Trp Tyr Asp Gly Ser Asn Lys Tyr Tyr Ala Asp Ser Val
        50                  55                  60
   Lys Gly Arg Phe Thr Ile Ser Arg Asp Asn Ser Lys Asn Thr Leu Tyr
    65                  70                  75                  80
   Leu Gln Met Asn Ser Leu Arg Ala Glu Asp Thr Ala Val Tyr Tyr Cys
                    85                  90                  95
   Ala Leu Met Val Arg Gly Leu Ile Ile Thr Gly Tyr Phe Asp Tyr Trp
               100                 105                 110
   Gly Gln Gly Thr Leu Val Thr Val Ser Ser
           115                 120
<210> 11
   <211> 324
   <212> DNA
   <213> Homo sapiens
<220>
   <221> CDS
   <222> (1)..(324)
<400> 11
   gaa att gtg ttg aca cag tct cca gcc acc ctg tct ttg tct cca ggg    48
   Glu Ile Val Leu Thr Gln Ser Pro Ala Thr Leu Ser Leu Ser Pro Gly
     1               5                  10                  15
   gaa aga gcc acc ctc tcc tgc agg gcc agt cag agt gtt agc agc tac    96
   Glu Arg Ala Thr Leu Ser Cys Arg Ala Ser Gln Ser Val Ser Ser Tyr
                 20                  25                  30
   tta gcc tgg tac caa cag aaa cct ggc cag gct ccc agg ctc ctc atc
   144
   Leu Ala Trp Tyr Gln Gln Lys Pro Gly Gln Ala Pro Arg Leu Leu Ile
             35                  40                  45
   tat gat gca tcc aac agg gcc act ggc atc cca gcc agg ttc agt ggc
   192
   Tyr Asp Ala Ser Asn Arg Ala Thr Gly Ile Pro Ala Arg Phe Ser Gly
         50                  55                  60
   agt ggg tct ggg aca gac ttc act ctc acc atc agc agc cta gag cct
   240
   Ser Gly Ser Gly Thr Asp Phe Thr Leu Thr Ile Ser Ser Leu Glu Pro
    65                  70                  75                  80
   gaa gat ttt gca gtt tat tac tgt cag cag cgt agc aac tgg cct ccg
   288
   Glu Asp Phe Ala Val Tyr Tyr Cys Gln Gln Arg Ser Asn Trp Pro Pro
                    85                  90                  95
   tac act ttt ggc cag ggg acc aag ctg gag atc aaa
   324
       Tyr Thr Phe Gly Gln Gly Thr Lys Leu Glu Ile Lys
                   100                 105
<210> 12
   <211> 108
   <212> PRT
   <213> Homo sapiens
<400> 12
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Arg Ser Asn Trp Pro Pro
   85 90 95
   Tyr Thr Phe Gly Gln Gly Thr Lys Leu Glu Ile Lys
   100 105
<210> 13
   <211> 3159
   <212> DNA
   <213> Artificial Sequence
<220>
   <223> Description of Artificial Sequence: cloning vector
<400> 13
   aattagcggc cgctgtcgac aagcttcgaa ttcagtatcg atgtggggta cctactgtcc 60
   cgggattgcg gatccgcgat gatatcgttg atcctcgagt gcggccgcag tatgcaaaaa 120
   aaagcccgct cattaggcgg gctcttggca gaacatatcc atcgcgtccg ccatctccag 180
   cagccgcacg cggcgcatct cgggcagcgt tgggtcctgg ccacgggtgc gcatgatcgt 240
   gctcctgtcg ttgaggaccc ggctaggctg gcggggttgc cttactggtt agcagaatga 300
   atcaccgata cgcgagcgaa cgtgaagcga ctgctgctgc aaaacgtctg cgacctgagc
   360
   aacaacatga atggtcttcg gtttccgtgt ttcgtaaagt ctggaaacgc ggaagtcagc
   420
   gccctgcacc attatgttcc ggatctgcat cgcaggatgc tgctggctac cctgtggaac
   480
   acctacatct gtattaacga agcgctggca ttgaccctga gtgatttttc tctggtcccg
   540
   ccgcatccat accgccagtt gtttaccctc acaacgttcc agtaaccggg catgttcatc
   600
   atcagtaacc cgtatcgtga gcatcctctc tcgtttcatc ggtatcatta cccccatgaa
   660
   cagaaattcc cccttacacg gaggcatcaa gtgaccaaac aggaaaaaac cgcccttaac
   720
   atggcccgct ttatcagaag ccagacatta acgcttctgg agaaactcaa cgagctggac
   780
   gcggatgaac aggcagacat ctgtgaatcg cttcacgacc acgctgatga gctttaccgc
   840
   agctgcctcg cgcgtttcgg tgatgacggt gaaaacctct gacacatgca gctcccggag
   900
   acggtcacag cttgtctgta agcggatgcc gggagcagac aagcccgtca gggcgcgtca
   960
   gcgggtgttg gcgggtgtcg gggcgcagcc atgacccagt cacgtagcga tagcggagtg
   1020
   tatactggct taactatgcg gcatcagagc agattgtact gagagtgcac catatgcggt
   1080
   gtgaaatacc gcacagatgc gtaaggagaa aataccgcat caggcgctct tccgcttcct
   1140
   cgctcactga ctcgctgcgc tcggtcgttc ggctgcggcg agcggtatca gctcactcaa
   1200
   aggcggtaat acggttatcc acagaatcag gggataacgc aggaaagaac atgtgagcaa
   1260
   aaggccagca aaaggccagg aaccgtaaaa aggccgcgtt gctggcgttt ttccataggc
   1320
   tccgcccccc tgacgagcat cacaaaaatc gacgctcaag tcagaggtgg cgaaacccga
   1380
   caggactata aagataccag gcgtttcccc ctggaagctc cctcgtgcgc tctcctgttc
   1440
   cgaccctgcc gcttaccgga tacctgtccg cctttctccc ttcgggaagc gtggcgcttt 1500
   ctcatagctc acgctgtagg tatctcagtt cggtgtaggt cgttcgctcc aagctgggct 1560
   gtgtgcacga accccccgtt cagcccgacc gctgcgcctt atccggtaac tatcgtcttg 1620
   agtccaaccc ggtaagacac gacttatcgc cactggcagc agccaggcgc gccttggcct 1680
   aagaggccac tggtaacagg attagcagag cgaggtatgt aggcggtgct acagagttct 1740
   tgaagtggtg gcctaactac ggctacacta gaaggacagt atttggtatc tgcgctctgc 1800
   tgaagccagt taccttcgga aaaagagttg gtagctcttg atccggcaaa caaaccaccg 1860
   ctggtagcgg tggttttttt gtttgcaagc agcagattac gcgcagaaaa aaaggatctc 1920
   aagaagatcc tttgatcttt tctacggggt ctgacgctca gtggaacgaa aactcacgtt 1980
   aagggatttt ggtcatgaga ttatcaaaaa ggatcttcac ctagatcctt ttaaattaaa 2040
   aatgaagttt taaatcaatc taaagtatat atgagtaaac ttggtctgac agttaccaat 2100
   gcttaatcag tgaggcacct atctcagcga tctgtctatt tcgttcatcc atagttgcct 2160
   gactccccgt cgtgtagata actacgatac gggagggctt accatctggc cccagtgctg 2220
   caatgatacc gcgagaccca cgctcaccgg ctccagattt atcagcaata aaccagccag 2280
   ccggaagggc cgagcgcaga agtggtcctg caactttatc cgcctccatc cagtctatta 2340
   attgttgccg ggaagctaga gtaagtagtt cgccagttaa tagtttgcgc aacgttgttg 2400
   ccattgctgc aggcatcgtg gtgtcacgct cgtcgtttgg tatggcttca ttcagctccg 2460
   gttcccaacg atcaaggcga gttacatgat cccccatgtt gtgcaaaaaa gcggttagct 2520
   ccttcggtcc tccgatcgtt gtcagaagta agttggccgc agtgttatca ctcatggtta 2580
   tggcagcact gcataattct cttactgtca tgccatccgt aagatgcttt tctgtgactg
   2640
   gtgagtactc aaccaagtca ttctgagaat agtgtatgcg gcgaccgagt tgctcttgcc
   2700
   cggcgtcaac acgggataat accgcgccac atagcagaac tttaaaagtg ctcatcattg
   2760
   gaaaacgttc ttcggggcga aaactctcaa ggatcttacc gctgttgaga tccagttcga
   2820
   tgtaacccac tcgtgcaccc aactgatctt cagcatcttt tactttcacc agcgtttctg
   2880
   ggtgagcaaa aacaggaagg caaaatgccg caaaaaaggg aataagggcg acacggaaat
   2940
   gttgaatact catactcttc ctttttcaat attattgaag catttatcag ggttattgtc
   3000
   tcatgagcgg atacatattt gaatgtattt agaaaaataa acaaataggg gttccgcgca
   3060
   catttccccg aaaagtgcca cctgacgtct aagaaaccat tattatcatg acattaacct
   3120
   ataaaaatag gcgtatcacg aggccctttc gtcttcaag
   3159